(19) Europäisches Patentamt
European Patent Office
Office européen des brevets

(11) Veröffentlichungsnummer: **0 407 899 B1**

(12) **EUROPÄISCHE PATENTSCHRIFT**

(45) Veröffentlichungstag der Patentschrift: **01.03.95**

(51) Int. Cl.[6]: **C07D 401/04**, A01N 43/54, C07D 401/14

(21) Anmeldenummer: **90112903.1**

(22) Anmeldetag: **06.07.90**

# (54) Aminopyrimidin-Derivate, Verfahren zu ihrer Herstellung, sie enthaltende Mittel und ihre Verwendung als Fungizide.

(30) Priorität: **11.07.89 DE 3922735**

(43) Veröffentlichungstag der Anmeldung:
**16.01.91 Patentblatt 91/03**

(45) Bekanntmachung des Hinweises auf die Patenterteilung:
**01.03.95 Patentblatt 95/09**

(84) Benannte Vertragsstaaten:
**DE FR GB IT**

(56) Entgegenhaltungen:
**EP-A- 0 270 362**
**US-A- 4 109 092**

**AUSTRALIAN JOURNAL OF CHEMISTRY, Band 35, Nr. 6, 1982, Seiten 1203-1207,Melbourne, AU; D.J. BROWN et al.: "Unfused heterobicycles as amplifiers of phleomycin. V. A range of pyridinylpyrimidines with strongly basic chains"**

**AUSTRALIAN JOURNAL OF CHEMISTRY, Band 33, Nr. 10, 1980, Seiten 2291-2298,Melbourne, AU; D.J. BROWN et al.: "Unfused heterobicycles as amplifiers ofphleomycin. I. Some pyridinyl- and pyrazolyl-pyrimidines, bithiazoles andthiazolylpyridines"**

(73) Patentinhaber: **Hoechst Schering AgrEvo GmbH**
**Gerichtstrasse 27**
**D-13342 Berlin (DE)**

(72) Erfinder: **Giencke, Wolfgang, Dr.**
**Am Steinberg 45**
**D-6238 Hofheim am Taunus (DE)**
Erfinder: **Sachse, Burkhard, Dr.**
**An der Ziegelei 30**
**D-6233 Kelkheim (Taunus) (DE)**
Erfinder: **Wicke, Heinrich, Dr.**
**Schillerstrasse 3**
**D-6239 Eppstein/Taunus (DE)**

EP 0 407 899 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Aminopyrimidin-Derivate, Verfahren zu ihrer Herstellung, sie enthaltende Mittel und ihre Verwendung als Fungizide.

Pyrimidin-Derivate sind bereits als wirksame Komponenten in fungiziden Mitteln bekannt (vgl. EP-A-270 362, EP-A-259 139, EP-A 234 104). Die Wirkung dieser Pyrimidin-Derivate ist jedoch insbesondere bei niedrigen Aufwandmengen nicht immer befriedigend.

Es wurden neue Pyrimidin-Derivate gefunden, die vorteilhafte Wirkungen bei der Bekämpfung eines breiten Spektrums phytopathogener Pilze insbesondere bei niedrigen Dosierungen aufweisen.

Gegenstand der vorliegenden Erfindung sind daher die Verbindungen der Formel I

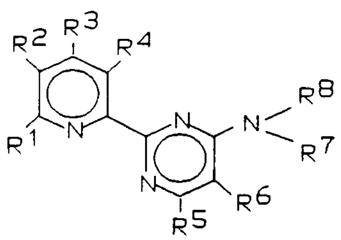

(I),

worin

$R^1$ = Wasserstoff, $(C_1$-$C_6)$Alkyl, $(C_1$-$C_4)$Alkoxy-$(C_1$-$C_4)$alkyl, $(C_1$-$C_4)$Alkylthio-$(C_1$-$C_4)$alkyl, $(C_2$-$C_6)$Alkenyl, $(C_2$-$C_6)$Alkinyl,$(C_3$-$C_7)$Cycloalkyl, $(C_3$-$C_7)$Cycloalkyl-$(C_1$-$C_4)$alkyl, wobei die beiden letztgenannten Reste im Cycloalkylteil bis zu dreifach durch $(C_1$-$C_4)$Alkyl substituiert sein können, eine Gruppe $R^7R^8N$-$(C_1$-$C_4)$alkyl, Phenyl, Phenoxy-$(C_1$-$C_4)$alkyl, Phenylmercapto-$(C_1$-$C_4)$alkyl, Phenyl-$(C_1$-$C_4)$alkyl, Phenoxy-phenoxy-$(C_1$-$C_4)$alkyl, wobei die fünf letztgenannten Reste im Phenylteil bis zu dreifach durch Halogen, Nitro, Cyano, $(C_1$-$C_4)$Alkyl, $(C_1$-$C_4)$Alkoxy, $(C_1$-$C_4)$Alkylthio, $(C_1$-$C_4)$Haloalkyl oder $(C_1$-$C_4)$-Haloalkoxy substituiert sein können,

$R^2$, $R^3$, $R^4$ = unabhängig voneinander Wasserstoff, $(C_1$-$C_6)$Alkyl, Phenyl, wobei der Phenylrest bis zu dreifach durch Halogen, Nitro, Cyano, $(C_1$-$C_4)$Alkyl, $(C_1$-$C_4)$Alkoxy, $(C_1$-$C_4)$Alkylthio, $(C_1$-$C_4)$Haloalkyl oder $(C_1$-$C_4)$Haloalkoxy substituiert sein kann,

$R^5$ = Wasserstoff, $(C_1$-$C_6)$Alkyl, $(C_3$-$C_7)$Cycloalkyl, $(C_3$-$C_7)$Cycloalkyl-$(C_1$-$C_4)$alkyl, wobei die beiden letztgenannten Reste im Cycloalkylteil bis zu dreifach durch $(C_1$-$C_4)$Alkyl substituiert sein können, $(C_1$-$C_4)$Haloalkyl, $(C_1$-$C_4)$Alkoxy, $(C_1$-$C_4)$Alkylthio, $(C_1$-$C_4)$-Alkoxy-$(C_1$-$C_4)$alkyl, eine Gruppe $R^7R^8N$-, $(C_1$-$C_4)$Alkylthio-$(C_1$-$C_4)$alkyl, eine Gruppe $R^7R^8N$-$(C_1$-$C_4)$alkyl, Halogen, $(C_2$-$C_6)$Alkenyl, $(C_2$-$C_6)$Alkinyl, Phenyl, Phenoxy, Phenyl-$(C_1$-$C_4)$alkyl, Phenoxy-$(C_1$-$C_4)$alkyl, Phenylmercapto-$(C_1$-$C_4)$alkyl, Phenylmercapto, Phenyl-$(C_1$-$C_4)$alkoxy oder Phenyl-$(C_1$-$C_4)$alkylthio, wobei die acht letztgenannten Reste im Phenylteil bis zu dreifach durch Halogen, Nitro, Cyano, $(C_1$-$C_4)$Alkyl, $(C_1$-$C_4)$Alkoxy, $(C_1$-$C_4)$Alkylthio, $(C_1$-$C_4)$Haloalkyl oder $(C_1$-$C_4)$Haloalkoxy substituiert sein können;

$R^6$ = Wasserstoff, $(C_1$-$C_4)$Alkyl, $(C_1$-$C_4)$Alkoxy, $(C_2$-$C_6)$Alkenyloxy, $(C_2$-$C_6)$Alkinyloxy, $(C_1$-$C_4)$Alkylthio, Halogen, Phenyl, wobei der Phenylrest bis zu dreifach durch Halogen, Nitro, Cyano, $(C_1$-$C_4)$Alkyl, $(C_1$-$C_4)$Alkoxy, $(C_1$-$C_4)$Alkylthio, $(C_1$-$C_4)$Haloalkyl oder $(C_1$-$C_4)$Haloalkoxy substituiert sein kann, oder

$R^5$ und $R^6$ bilden zusammen eine Polymethylenkette der Formel $-(CH_2)_m-$ mit m = 3 - 4 und

$R^7$ $(C_1$-$C_6)$Alkyl, $(C_1$-$C_4)$Alkoxy-$(C_1$-$C_6)$-Alkyl, Hydroxy-$(C_1$-$C_6)$Alkyl, $(C_1$-$C_4)$Alkylthio-$(C_1$-$C_6)$Alkyl, $R^9R^{10}N$-$(C_1$-$C_6)$Alkyl, $(C_3$-$C_6)$Alkenyl, $(C_3$-$C_6)$Alkinyl, $(C_3$-$C_7)$Cycloalkyl, $(C_3$-$C_7)$Cycloalkyl-$(C_1$-$C_4)$alkyl, wobei die beiden letztgenannten Reste im Cycloalkylteil bis zu dreifach durch $(C_1$-$C_4)$Alkyl substituiert sein können; Formyl, Phenyl, Phenyl-$(C_1$-$C_4)$alkyl, wobei die beiden letztgenannten Reste im Phenylteil bis zu dreifach durch Halogen, Nitro, Cyano, $(C_1$-$C_4)$Alkyl, $(C_1$-$C_4)$Alkoxy, $(C_1$-$C_4)$Alkylthio, $(C_1$-$C_4)$Haloalkyl oder $(C_1$-$C_4)$Haloalkoxy substituiert sein können;

$R^8$ Wasserstoff bedeutet oder wie $R^7$ definiert ist;

oder beide Reste $R^7$, $R^8$ stehen zusammen mit dem Stickstoffatom, an das sie gebunden sind, für einen unsubstituierten oder bis zu vierfach mit $(C_1$-$C_4)$-Alkyl substituierten 5- bis 7-gliedrigen, gesättigten oder ungesättigten Heterocyclus mit 1 bis

3 gleichen oder verschiedenen Heteroatomen, worin die Heteroatome Stickstoff, Sauerstoff und/oder Schwefel stehen;

$R^9$, $R^{10}$ = unabhängig voneinander Wasserstoff, $(C_1-C_6)$Alkyl, $(C_3-C_6)$Alkenyl, $(C_3-C_6)$Alkinyl, $(C_3-C_7)$Cycloalkyl, $(C_3-C_7)$Cycloalkyl-$(C_1-C_4)$alkyl, wobei die beiden letztgenannten Reste im. Cycloalkylteil bis zu dreifach durch $(C_1-C_4)$Alkyl substituiert sein können; Formyl, Phenyl, Phenyl$(C_1-C_4)$alkyl, wobei die beiden letztgenannten Reste im Phenylteil bis zu dreifach durch Halogen, Nitro, Cyano, $(C_1-C_4)$Alkyl, $(C_1-C_4)$Alkoxy, $(C_1-C_4)$Alkylthio, $(C_1-C_4)$Haloalkyl oder $(C_1-C_4)$Haloalkoxy substituiert sein können;

oder beide Reste $R^9$, $R^{10}$ stehen zusammen mit dem Stickstoffatom, an das sie gebunden sind, für einen unsubstituierten oder bis zu vierfach mit $(C_1-C_4)$-Alkyl substituierten 5- bis 7-gliedrigen, gesättigten oder ungesättigten Heterocyclus mit 1 bis 3 gleichen oder verschiedenen Heteroatomen, worin die Heteroatome Stickstoff, Sauerstoff und/oder Schwefel stehen; bedeuten, sowie deren Säureadditionssalze; mit der Maßgabe, daß

die Reste $R^1$-$R^8$ nicht gleichzeitig folgende Bedeutungen haben:

a) $R^1$, $R^2$, $R^3$, $R^4$, $R^6$ und $R^8$ jeweils = Wasserstoff, $R^5$ = Methyl, und $R^7$ = 2-(N,N-Dimethylamino)-ethyl oder 3-(N,N-Dimethylamino)-propyl; oder

b) $R^1$, $R^2$, $R^3$, $R^4$ und $R^6$ jeweils = Wasserstoff, $R^7$ und $R^8$ jeweils = Methyl, und $R^5$ = Chlor oder Ethylmercapto.

Dabei können die Alkyl, Alkenyl- oder Alkinylreste sowohl geradkettig als auch verzweigt sein. Halogen bedeutet F, Cl, Br, J, bevorzugt F, Cl und Br. Die Vorsilbe "Halo" in der Bezeichnung eines Substituenten bedeutet hier und im folgenden, daß dieser Substituent einfach oder mehrfach bei gleicher oder verschiedener Bedeutung auftreten kann. Die Vorsilbe "Halo" beinhaltet Fluor, Chlor, Brom oder Jod, insbesondere Fluor, Chlor oder Brom. Als Beispiele für Halogenalkyl seien genannt: $CF_3$, $CF_2CHF_2$, $CF_2CF_3$, $CCl_3$, $CCl_2F$, $CF_2CF_2CF_3$, $CF_2CHFCF_3$ und $(CF_2)_3CF_3$. Beispiele für Haloalkoxy sind $OCF_3$, $OCF_2CHF_2$ oder $OCF_2CF_2CF_3$.

Bevorzugt unter den Verbindungen der Formel I sind solche, worin

$R^1$ = Wasserstoff, $(C_1-C_6)$Alkyl, Phenyl, Phenyl-$(C_1-C_2)$alkyl, Phenoxy-phenoxy-$(C_1-C_2)$alkyl, Phenoxy-$(C_1-C_2)$alkyl, wobei die vier letztgenannten Reste im Phenylteil bis zu dreifach durch Halogen oder $(C_1-C_4)$Alkyl substituiert sein können; $(C_1-C_3)$Alkoxy-$(C_1-C_2)$alkyl,

$R^2$, $R^3$ = unabhängig voneinander Wasserstoff, $(C_1-C_3)$Alkyl, Phenyl, wobei der Phenylrest bis zu dreifach durch Halogen oder $(C_1-C_4)$Alkyl substituiert sein kann,

$R^4$ = Wasserstoff,

$R^5$ = Wasserstoff, $(C_1-C_6)$Alkyl, $(C_3-C_6)$Cycloalkyl, $(C_5-C_6)$Cycloalkyl-$(C_1-C_3)$alkyl, Halogen, Phenyl, Phenyl-$(C_1-C_2)$alkyl, wobei die beiden letztgenannten Reste im Phenylteil unsubstituiert oder bis zu dreifach durch Halogen, $(C_1-C_4)$Alkyl oder $(C_1-C_4)$Alkoxy substituiert sein können,

$R^6$ = Wasserstoff, $(C_1-C_4)$Alkyl, Halogen, Phenyl, $(C_1-C_3)$Alkoxy oder

$R^5$ und $R^6$ bilden zusammen eine Polymethylenkette der Formel $-(CH_2)_m-$ mit m = 3 - 4 und

$R^7$ $(C_1-C_6)$Alkyl, $(C_1-C_4)$Alkoxy-$(C_1-C_6)$-Alkyl, Hydroxy-$(C_1-C_6)$Alkyl, $(C_1-C_4)$Alkylthio-$(C_1-C_6)$-Alkyl, $R^9R^{10}N$-$(C_1-C_6)$Alkyl, $(C_3-C_4)$Alkenyl, $(C_3-C_4)$Alkinyl, $(C_3-C_6)$Cycloalkyl, $(C_3-C_6)$-Cycloalkyl-$(C_1-C_3)$alkyl, wobei die beiden letztgenannten Reste im Cycloalkylteil bis zu zweifach durch $(C_1-C_2)$Alkyl substituiert sein können; Formyl, Phenyl, Phenyl-$(C_1-C_2)$alkyl, wobei die beiden letztgenannten Reste im Phenylteil bis zu zweifach durch Halogen, $(C_1-C_3)$Alkyl, $(C_1-C_3)$Alkoxy, Trifluormethyl oder Trichlormethyl substituiert sein können; bedeuten;

$R^8$ Wasserstoff bedeutet oder wie $R^7$ definiert ist, oder

beide Reste $R^7$, $R^8$ stehen zusammen mit dem Stickstoffatom, an das sie gebunden sind, für einen unsubstituierten oder bis zu zweifach mit $(C_1-C_3)$-Alkyl substituierten 5- bis 7-gliedrigen gesättigten oder ungesättigten Heterocyclus mit 1 oder 2 gleichen oder verschiedenen Heteroatomen Stickstoff und/oder Sauerstoff;

$R^9$, $R^{10}$ = unabhängig voneinander Wasserstoff, $(C_1-C_6)$Alkyl, $(C_3-C_6)$Alkenyl, $(C_3-C_6)$Alkinyl, $(C_3-C_7)$Cycloalkyl, $(C_3-C_7)$Cycloalkyl-$(C_1-C_4)$alkyl, wobei die beiden letztgenannten Reste im Cycloalkylteil bis zu dreifach durch $(C_1-C_4)$Alkyl substituiert sein können; Formyl, Phenyl, Phenyl$(C_1-C_4)$alkyl, wobei die beiden letztgenannten Reste im Phenylteil bis zu dreifach durch Halogen, Nitro, Cyano, $(C_1-C_4)$Alkyl, $(C_1-C_4)$Alkoxy, $(C_1-C_4)$Alkylthio, $(C_1-C_4)$-Haloalkyl oder $(C_1-C_4)$Haloalkoxy substituiert sein können;

oder beide Reste $R^9$, $R^{10}$ stehen zusammen mit dem Stickstoffatom, an das sie

gebunden sind, für einen unsubstituierten oder bis zu vierfach mit ($C_1$-$C_4$)-Alkyl substituierten 5- bis 7-gliedrigen, gesättigten oder ungesättigten Heterocyclus mit 1 bis 3 gleichen oder verschiedenen Heteroatomen Stickstoff, Sauerstoff und/oder Schwefel; bedeuten, sowie deren Säureadditionssalze.

Zur Herstellung der Säureadditionssalze der Verbindungen der Formel I kommen folgende Säuren in Frage: Halogenwasserstoffsäuren wie Chlorwasserstoffsäure oder Bromwasserstoffsäure, ferner Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphthalindisulfonsäure. Die Säureadditionssalze der Verbindungen der Formel I können in einfacher Weise nach üblichen Salzbildungsmethoden, z. B. durch Lösen einer Verbindung der Formel I in einem geeigneten organischen Lösemittel und Hinzufügen der Säure erhalten werden und in bekannter Weise, z. B. durch Abfiltrieren, isoliert und gegebenenfalls durch Waschen mit einem inerten organischen Lösemittel gereinigt werden.

Gegenstand der vorliegenden Erfindung ist auch ein Verfahren zur Herstellung der Verbindungen der Formel I, dadurch gekennzeichnet, daß man eine Verbindung der Formel II in Gegenwart einer Base mit einer Verbindung der Formel III umsetzt.

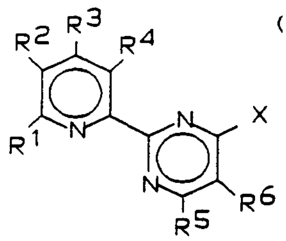
(II)

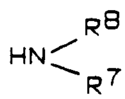
(III)

Die Substituenten $R^1$ bis $R^8$ haben dabei die Bedeutungen wie in der Formel I. X steht für Halogen. Halogen bedeutet Fluor, Chlor, Brom oder Jod, insbesondere Chlor oder Brom.

Die Umsetzung der Verbindungen II mit III erfolgt vorzugsweise in inerten aprotischen Lösungsmitteln wie z. B. Acetonitril, Dichlormethan, Toluol, Xylol, Tetrahydrofuran, Dioxan, Dialkylether wie Diethylenglykoldialkylether, insbesondere Diethylenglykoldiethylether, oder DMF bei Temperaturen zwischen -10 °C und der Siedetemperatur des Lösungsmittels. Als Basen eignen sich die für diesen Reaktionstyp üblichen Basen wie beispielsweise Carbonate und Hydrogencarbonate von Alkali- und Erdalkalimetallen, Alkalihydroxide, Alkalialkoholate wie K-tert.-butylat, tert.-Amine, Pyridin oder substituierte Pyridinbasen (z. B. 4-Dimethylaminopyridin).

Auch ein zweites Äquivalent der Verbindungen der allgemeinen Formel III kann die Funktion der Base übernehmen.

Die Verbindungen der Formel II können nach bekannten Verfahren hergestellt werden (vgl. EP-A-234 104, EP-A-259 139, EP-A-270 362, J. Org. Chem. 32, 1591, (1967)). Die Verbindungen der Formel III sind bekannt und leicht zugänglich (Houben-Weyl, Methoden der Org. Chemie, Band XI/1).

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich bedeutender, phytopathogener Pilze, wie z.B. Piricularia oryzae, Venturia inaequalis, Cercospora beticola, Echte Mehltauarten, Fusariumarten, Plasmopora viticola, Pseudoperonospra cubensis, verschiedene Rostpilze und Pseudocercosporella herpotrichoides. Besonders gut werden Benzimidazol- und Dicarboximid-sensible und -resistente Boytritis cinerea-Stämme erfaßt.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Gegenstand der Erfindung sind auch Mittel, die die Verbindungen der Formel I neben geeigneten Formulierungshilfsmitteln enthalten.

Die erfindungsgemäßen Mittel enthalten die Wirkstoffe der Formel I im allgemeinen zu 1 bis 95 Gew.-%.

Sie können auf verschiedene Art formuliert werden, je nachdem wie es durch die biologischen und/oder chemischphysikalischen Parameter vorgegeben ist. Als Formulierungsmöglichkeiten kommen daher in Frage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C-Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs-oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2′-dinaphthylmethan-6,6′-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Poryphillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierugen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, sind z.B. folgende Produkte zu nennen:

Imazalil, Prochloraz, Fenapanil, SSF 105, Triflumizol, PP 969, Flutriafol, BAY-MEB 6401, Propiconazol, Etaconazol, Diclobutrazol, Bitertanol, Triadimefon, Triadimenol, Fluotrimazol, Tridemorph, Dodemorph, Fenpropimorph, Falimorph, S-32165, Chlobenzthiazone, Parinol, Buthiobat, Fenpropidin, Triforine, Fenarimol, Nuarimol, Triarimol, Ethirimol, Dimethirimol,
Bupirimate, Rabenzazole, Tricyclazole, Fluobenzimine, Pyroxyfur, NK-483, PP-389, Pyroguilon, Hymexazole, Fenitropan, UHF-8227, Cymoxanil, Dichlorunanid, Captafol, Captan, Folpet, Tolylfluanid, Chlorothalonil, Etridiazol, Iprodione (Formel II), Procymidon, Vinclozolin, Metomeclan, Myclozolin, Dichlozolinate, Fluorimide, Drazoxolan, Chinomethionate, Nitrothalisopropyl, Dithianon, Dinocap, Binapacryl,
Fentinacetate, Fentinhydroxide, Carboxin, Oxycarboxin, Pyracarbolid, Methfuroxam, Fenfura, Furmecyclox, Benodanil, Mebenil, Mepronil, Flutalanil, Fuberidazole, Thiabendazole, Carbendazim, Benomyl, Thiofante, Thiofanatemethyl, CGD-94340 F, IKF-1216,
Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cuoxychlorid, Oxine-Cu, Cu-oxide, Schwefel, Fosetylaluminium, Natrium-dodecylbenzolsulfonat,
Natrium-dodecylsulfat,
Natrium-C13/C15-alkoholethersulfonat,
Natrium-cetostearylphosphatester,
Dioctyl-natriumsulfosuccinat,
Natrium-isopropylnaphthalinsulfonat,
Natrium-methylenbisnaphthalinsulfonat,
Cetyl-trimethyl-ammoniumchlorid,
Salze von langkettigen primären, sekundären oder tertiären Aminen, Alkyl-propylenamine, Lauryl-pyridinium-bromid, ethoxilierte quaternierte Fettamine, Alkyl-dimethyl-benzyl-ammoniumchlorid und 1 Hydroxyethyl-2-alkyl-imidazolin.

Die oben genannten Kombinationspartner stellen bekannte Wirkstoffe dar, die zum großen Teil in CH.R. Worthing, U.S.B. Walker, The Pesticide Manual, 7. Auflage (1983), British Crop Protection Council beschrieben sind.

Darüberhinaus können die erfindungsgemäßen Wirkstoffe, insbesondere die der aufgeführten Beispiele, in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a.. Bevorzugte Mischungspartner sind:

1. aus der Gruppe der Phosphorsäureester
Azinphos-ethyl, Azinphos-methyl, 1-(4-Chlorphenyl)-4-(O-ethyl, S-propyl)phosphoryloxypyrazol (TIA-230), Chlorpyrifos, Coumaphos, Demeton, Demeton-S-methyl, Diazinon, Dichlorvos, Dimethoat, Ethoprophos, Etrimfos, Fenitrothion, Fenthion, Heptenophos, Parathion, Parathion-methyl, Phosalon, Pirimiphos-ethyl, Pirimiphos-methyl, Profenofos, Prothiofos, Sulprofos, Triazophos, Trichlorphon.

2. aus der Gruppe der Carbamate
Aldicarb, Bendiocarb, BPMC (2-(1-Methylpropyl)phenyl methylcarbamat), Butocarboxim, Butoxicarboxim, Carbaryl, Carbofuran, Carbosulfan, Cloethocarb, Isoprocarb, Methomyl, Oxamyl, Primicarb, Promecarb, Propoxur, Thiodicarb.

3. aus der Gruppe der Carbonsäureester
Allethrin, Alphamethrin, Bioallethrin, Bioresmethrin, Cycloprothrin, Cyfluthrin, Cyhalothrin, Cypermethrin, Deltamethrin, 2,2-Dimethyl-3-(2-chlor-2-trifluormethylvinyl)cyclopropancarbonsäure-(alpha-cyano-3-phenyl-2-methyl-benzyl)ester (FMC 54800), Fenpropathrin, Fenfluthrin, Fenvalerat, Flucythrinate, Flumethrin, Fluvalinate, Permethrin, Resmethrin, Tralomethrin.

4. aus der Gruppe der Formamidine
Amitraz, Chlordimeform

5. aus der Gruppe der Zinnverbindungen
Azocyclotin, Cyhexatin, Fenbutatinoxid

6. Sonstige
Abamektin, Bacillus thuringiensis, Bensultap, Binapacryl, Bromopropylate, Buprofecin, Camphechlor, Cartap, Chlorbenzialate, Chlorfluazuron, 2-(4-Chlorphenyl)-4,5-diphenylthiophen (UBI-T 930), Chlofentezine, Cyclopropancarbonsäure(2-naphthylmethyl)ester (Ro 12-0470), Cyromacin, DDT, Dicofol, N-(3,5-Dichlor-4-(1,1,2,2,-tetrafluoroethoxy)phenylamino)carbonyl)-2,6-difluorbenzamide (XRD 473), Diflubenzuron, N-(2,3-Dihydro-3-methyl-1,3-thiazol-2-ylidene)2,4-xylidine, Dinobuton, Dinocap, Endosulfan, Fenoxycarb, Fenthiocarb, Flubenzimine, Flufenoxuron, Gamma-HCH, Hexythiazox, Hydramethylnon (AC 217

300) Ivermectin, 2-Nitromethyl-4,5-dihydro-6H-thiazin (SD 52618), 2-Nitromethyl-3,4-dihydrothiazol (SD 35651), 2-Nitromethylene-1,3-thiazinan-3-yl-carbamaldehyde (WL 108 477), Propargite, Teflubenzuron, Tetradifon, Tetrasul, Thiocyclam, Triflumaron, Kernpolyeder- und Granuloseviren.

Der Wirkstoffgehalt der aus den handelsüblichen Formulierungen bereiteten Anwendungsformen kann in weiten Bereichen variieren, Die Wirkstoffkonznetration der Anwendungsformen kann von 0,0001 bis zu 100 Gew.-% Wirkstoff, vorzugsweise zwischen 0,001 und 1 Gew.-% liegen. Die Anwendung geschieht in einer den Anwendungsformen angepaßten üblichen Weisen.

Nachfolgende Beispiele dienen zur Erläuterung der Erfindung.

## A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

## B. Chemische Beispiele

### 4-Methyl-2-(2-methyl-pyridin-6-yl)-6-propylamino-pyrimidin (Bsp. Nr. 1.2)

Zu einer Lösung von 1,10 g (5 mmol) 4-Chlor-6-methyl-2-(2-methyl-pyridin-6-yl)-pyrimidin in 30 ml Acetonitril fügt man nacheinander 0,32 g (5,5 mmol) Propylamin, 0,83 g (6 mmol) $K_2CO_3$ und 10 mg Benzyltriethylammoniumchlorid hinzu. Die Reaktionsmischung wird 7 h am Rücklfuß gekocht. Danach saugt man alle unlöslichen Bestandteile ab. Das Filtrat wird eingeengt, in Methylenchlorid gelöst, anschließend mit Wasser gewaschen, über $Na_2SO_4$ getrocknetund im Vakuum eingedampft. Man erhält 1,15 g (95 %), der Titelverbindung als gelbliches Öl.

### 4-Chlor-6-diethylamino-2-(2-methyl-pyridin-6-yl)-pyrimidin (Bsp. 9.5)

Zu einer Lösung von 1,44 g (6 mmol) 4,6-Dichlor-2-(2-methyl-pyridin-6-yl)-pyrimidin in 30 ml Acetonitril fügt man nacheinander 0,48 g (6,6 mmol) Diethylamin, 0,97 g (7,0 mmol) $K_2CO_3$ und 10 mg Benzyltriethylammoniumchlorid. Die Reaktionsmischung wird 3 h bei Raumtemperatur gerührt.

Danach saugt man alle unlöslichen Bestandteile ab. Das Filtrat wird eingeengt, in Methylenchlorid gelöst, mit Wasser gewaschen, über $Na_2SO_4$ getrocknet und im Vakuum eingedampft. Man erhält 1,73 g (92 %) der Titelverbindung als grünliches Öl.

### 4-Phenyl-6-propylamino-2-(2-methylpyridin-6-yl)-pyrimidin Hydrochlorid (Bsp. Nr. 200.1)

In eine Lösung von 3,4 g (0,01 mol) 4-Phenyl-6-propylamino-2-(2-methylpyridin-6-yl)-pyrimidin leitet man über einen Zeitraum von 1 h HCl-Gas ein. Der ausgefallene Feststoff wird abgesaugt. Er zerfließt sofort zu einer sirupösen Masse.

Analog zu diesen Beispielen lassen sich die Verbindungen der Tabellen A und B herstellen.

Abkürzungen: Et = Ethyl
Me = Methyl
Pr = Propyl

**Tabelle A**

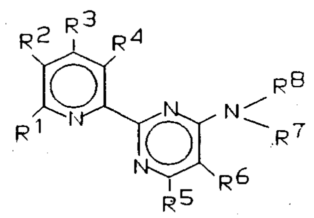

(I)

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 1.1 | $CH_3$ | H | H | H | $CH_3$ | H | $NEt_2$ | $^1H$-NMR ($CDCl_3$): d 8,16 t 7,66 d 7,20 s 6,21 q 3,57 s 2,69 d 2,48 t 1,19 [ppm] |
| 1.2 | $CH_3$ | H | H | H | $CH_3$ | H | NH Propyl | $^1H$-NMR ($CDCl_3$): d 8,19 t 7,70 d 7,19 s 6,22 q 3,26 s 2,67 s 2,45 dq 1,57 t 0,99 [ppm] |
| 1.3 | $CH_3$ | H | H | H | $CH_3$ | H | $NMe_2$ | $^1H$-NMR ($CDCl_3$): d 8,17 t 7,54 d 7,22 s 3,16 s 2,69 s 2,49 [ppm] |
| 1.4 | $CH_3$ | H | H | H | $CH_3$ | H | $NCH_3C_6H_5$ | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 1.5 | $CH_3$ | H | H | H | $CH_3$ | H | NHMe | |
| 1.6 | $CH_3$ | H | H | H | $CH_3$ | H | NHEt | |
| 1.7 | $CH_3$ | H | H | H | $CH_3$ | H | 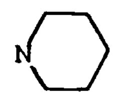 | |
| 1.8 | $CH_3$ | H | H | H | $CH_3$ | H | $NHC_6H_5$ | |
| 1.9 | $CH_3$ | H | H | H | $CH_3$ | H | $NCH_2C_6H_5$ | |
| 1.10 | $CH_3$ | H | H | H | $CH_3$ | H | $NHC_6H_4$-4-Cl | |

Tabelle A Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.1 | $CH_3$ | H | H | H | $C_3H_7$ | H | $NEt_2$ | $^1H$-NMR ($CDCl_3$): d 8,14 t 7,65 d 7,19 s 6,22 q 3,69 m 2,69 s 2,65 dq 1,79 t 1,21 t 1,03 [ppm] |
| 2.2 | $CH_3$ | H | H | H | $C_3H_7$ | H | NH Propyl | $^1H$-NMR ($CDCl_3$): d 8,17 t 7,66 d 7,16 s 6,17 q 3,25 m 2,69 s 2,64 m 1,68 t 1,00 t 0,98 [ppm] |
| 2.3 | $CH_3$ | H | H | H | $C_3H_7$ | H | 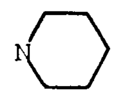 | $^1H$-NMR ($CDCl_3$): d 8,14 t 7,66 d 7,20 s 6,34 m 3,70 m 2,80 s 2,75 m 1,64 t 0,98 [ppm] |
| 2.4 | $CH_3$ | H | H | H | $C_3H_7$ | H | 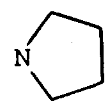 | $^1H$-NMR ($CDCl_3$): d 8,15 t 7,65 t 7,19 s 6,11 m 3,56 t 2,72 s 2,69 m 2,00 m 1,74 t 1,0 [ppm] |
| 2.5 | $CH_3$ | H | H | H | $C_3H_7$ | H | 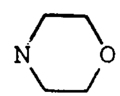 | $^1H$-NMR ($CDCl_3$): d 8,16 t 7,68 d 7,20 s 6,84 m 3,77 m 2,75 s 2,66 m 1,78 t 0,99 [ppm] |
| 2.6 | $CH_3$ | H | H | H | $C_3H_7$ | H | $NMe_2$ | $^1H$-NMR (DMSO-$d_6$): 8,07 t 7,68 d 7,29 s 6,52 s 3,11 t 2,59 s 2,54 m 1,79 t 0,95 [ppm] |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.7 | $CH_3$ | H | H | H | $C_3H_7$ | H | NHMe | $^1H$-NMR ($CDCl_3$): d 8,16 t 7,67 d 7,16 s 6,19 m 5,41 d 2,94 t 2,66 s 2,63 m 1,79 t 0,97 [ppm] |
| 2.8 | $CH_3$ | H | H | H | $C_3H_7$ | H | $NCH_3C_6H_5$ | Smp.: 145°C |
| 2.9 | $CH_3$ | H | H | H | $C_3H_7$ | H | NHEt | Smp.: 100 - 102°C |
| 2.10 | $CH_3$ | H | H | H | $C_3H_7$ | H | $NHCH_2C{\equiv}CH$ | |
| 2.11 | $CH_3$ | H | H | H | $C_3H_7$ | H | $NHCH_2-CH=CH_2$ | Smp.: 111 - 113°C |
| 2.12 | $CH_3$ | H | H | H | $C_3H_7$ | H | NH Heptyl | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.13 | $CH_3$ | H | H | H | $C_3H_7$ | H | NH Butyl | Smp.: 99°C |
| 2.14 | $CH_3$ | H | H | H | $C_3H_7$ | H | NH iso-Butyl | Smp.: 119°C |
| 2.15 | $CH_3$ | H | H | H | $C_3H_7$ | H | NH sec-Butyl | Smp.: 107°C |
| 2.16 | $CH_3$ | H | H | H | $C_3H_7$ | H | NH Pentyl | Smp.: 76°C |
| 2.17 | $CH_3$ | H | H | H | $C_3H_7$ | H | NH Benzyl | 1-NMR ($CDCl_3$): d 8,19 t 7,67 m 7,36-7,21 d 7,16 s 6,16 t 5,63 d 4,55 t 2,69 s 2,67 dq 1,75 t 0,98 [ppm] |
| 2.18 | $CH_3$ | H | H | H | $C_3H_7$ | H | NMe Benzyl | $^1$H-NMR ($CDCl_3$): d 8,13 t 7,62 s 7,27 d 7,15 s 6,30 s 4,88 s 3,11 t 2,72 s 2,69 dq 1,77 t 0,98 [ppm] |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.19 | $CH_3$ | H | H | H | $C_3H_7$ | H | NH iso-Propyl | Smp.: 118 - 120°C |
| 2.20 | $CH_3$ | H | H | H | $C_3H_7$ | H | NH Cyclohexyl | Smp.: 90 - 92°C |
| 2.21 | $CH_3$ | H | H | H | $C_3H_7$ | H | NH Cyclopentyl | Smp.: 146°C |
| 2.22 | $CH_3$ | H | H | H | $C_3H_7$ | H | NH $C_6H_5$ | |
| 2.23 | $CH_3$ | H | H | H | $C_3H_7$ | H | NH (4-Cl-$C_6H_4$) | Smp.: 103 - 105°C |
| 2.24 | $CH_3$ | H | H | H | $C_3H_7$ | H | NH (2,4 $Cl_2$-$C_6H_3$) | |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.25 | $CH_3$ | H | H | H | $C_3H_7$ | H | NH (4-$CH_3$-$C_6H_4$) | |
| 2.26 | $CH_3$ | H | H | H | $C_3H_7$ | H | NH (4-$NO_2$-$C_6H_4$) | |
| 2.27 | $CH_3$ | H | H | H | $C_3H_7$ | H | NH (3-$CH_3$-$C_6H_4$) | |
| 2.28 | $CH_3$ | H | H | H | $C_3H_7$ | H | NH-Cyclopropyl | |
| 2.29 | $CH_3$ | H | H | H | $C_3H_7$ | H | NH-$CH_2CH$=C(Me)$_2$ | |
| 2.30 | $CH_3$ | H | H | H | $C_3H_7$ | H | NH-$C_6H_4$-4-OMe | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.31 | $CH_3$ | H | H | H | $C_3H_7$ | H | NH-$C_6H_4$-3$CF_3$ | |
| 2.32 | $CH_3$ | H | H | H | $CH(CH_3)_2$ | H | $NEt_2$ | |
| 2.33 | $CH_3$ | H | H | H | $C_3H_7$ | H | 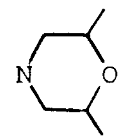 | Smp.: 151°C |
| 2.34 | $CH_3$ | H | H | H | $CH(CH_3)_2$ | H | NH-Propyl | Smp.: 105°C |
| 2.35 | $CH_3$ | H | H | H | $CH(CH_3)_2$ | H | NH-Butyl | |
| 2.36 | $CH_3$ | H | H | H | $CH(CH_3)_2$ | H | NH-Pentyl | |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.37 | $CH_3$ | H | H | H | $CH(CH_3)_2$ | H | 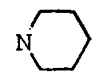 | |
| 2.38 | $CH_3$ | H | H | H | $C_3H_7$ | H | 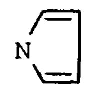 | Smp. 113°C |
| 2.39 | $CH_3$ | H | H | H | $C_3H_7$ | H | $NCH_3CH_2$-$C_6H_5$ | |
| 2.40 | $CH_3$ | H | H | H | $C_3H_7$ | H | 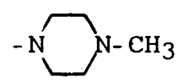 | $^1$H-NMR ($CDCl_3$): d 8,16 t 7,66 d 7,19 s 6,36 t 3,76 t 2,73 s 2,71 t 2,50 s 2,36 dq 1,76 t 1,00 [ppm] |
| 2.41 | $CH_3$ | H | H | H | $CH(CH_3)_2$ | H | 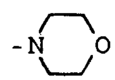 | $^1$H-NMR ($CDCl_3$): d 8,19 t 7,69 d 7,19 s 6,35 m 3,86-3,70 sep 3,05 s 2,69 d 1,31 [ppm] |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.42 | $CH_3$ | H | H | H | $CH(CH_3)_2$ | H | 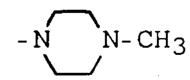 | Smp.: 105°C |
| 2.43 | $CH_3$ | H | H | H | $CH(CH_3)_2$ | H | $NH-C_6H_4-4-Cl$ | Smp.: 128 - 129°C |
| 2.44 | $CH_3$ | H | H | H | $C_3H_7$ | H | 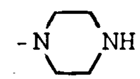 | Smp.: 180°C |
| 2.45 | $CH_3$ | H | H | H | $C_5H_9$ | H | 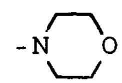 | Smp.: 143-144°C |
| 2.46 | $CH_3$ | H | H | H | $C_5H_9$ | H | 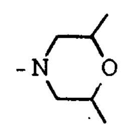 | Smp.: 162°C |
| 2.47 | $CH_3$ | H | H | H | $C_5H_9$ | H | $NHCHCH_3C_2H_5$ | Smp.: 99 - 102°C |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.48 | $CH_3$ | H | H | H | $C_5H_9$ | H | $NHCH_2C_6H_5$ | Smp.: 111°C |
| 2.49 | $CH_3$ | H | H | H | $C_5H_9$ | H | NH - 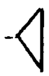 | Smp.: 124 - 126°C |
| 2.50 | $CH_3$ | H | H | H | $C_5H_9$ | H | $NHCH_2CH=CH_2$ | Smp.: 133°C |
| 2.51 | $CH_3$ | H | H | H | $C_5H_9$ | H | 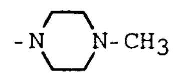 | $^1$H-NMR($CDCl_3$) d 8,15 t 7,66 d 7,21 s 6,36 t 3,77 m 2,90-2,67 s 2,69 t 2,50 s 2,31 m 1,90-1,59 m 1,52-1,20 t 0,85 [ppm] |
| 2.52 | $CH_3$ | H | H | H | $CH_3$ | Cl | 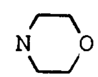 | Smp.: 72 - 74°C |
| 2.53 | $CH_3$ | H | H | H | $CH_3$ | Cl | 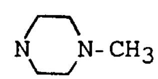 | Smp.: 80-83°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 2.54 | $CH_3$ | H | H | H | $CH_3$ | Cl | 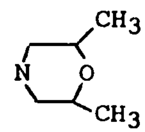 | Smp.: 95 - 97°C |
| 2.55 | $CH_3$ | H | H | H | $C_3H_7$ | H | $NHCH_2CH_2N(CH_3)_2$ | $^1H$-NMR ($CDCl_3$): d 8,19 t 7,66 d 7,19 s 6,16 s 2,69 m 1,78 t 0,99 |
| 2.56 | $CH_3$ | H | H | H | $CH_3$ | H | $NHCH_2CH_2OH$ | |
| 2.57 | $CH_3$ | H | H | H | $CH_3$ | Cl | $NHCH_2CH_2OCH_3$ | |
| 2.58 | $CH_3$ | H | H | H | $(CH_3)_2CH$ | H | $NCH_2CH_2N$ 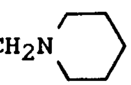 | |
| 2.59 | $CH_3$ | H | H | H | $C_4H_9$ | Br | $NHCH_2CH_2SCH_3$ | |
| 3.1 | $CH_3$ | H | H | H | $C_6H_5$ | H | NHMe | $^1H$-NMR ($CDCl_3$): d 8,41 m 8,14 t 7,71 m 7,47 d 7,21 s 6,70 s 3,04 s 2,72 [ppm] |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 3.2 | $CH_3$ | H | H | H | $C_6H_5$ | H | $NEt_2$ | $^1H$-NMR ($CDCl_3$): d 8,26 m 8,10 t 7,69 m 7,45 d 7,20 s 6,75 q 3,69 s 2,71 t 1,27 [ppm] |
| 3.3 | $CH_3$ | H | H | H | $C_6H_5$ | H | 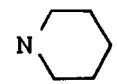 | Smp.: 120 - 122°C |
| 3.4 | $CH_3$ | H | H | H | $C_6H_5$ | H | NHBu | Smp.: 119 - 121°C |
| 3.5 | $CH_3$ | H | H | H | $C_6H_5$ | H | NHPr | Smp.: 127 - 129°C |
| 3.6 | $CH_3$ | H | H | H | $C_6H_5$ | H | NHiso-Propyl | Smp.: 105°C |
| 3.7 | $CH_3$ | H | H | H | $C_6H_5$ | H | 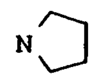 | Smp.: 134°C |
| 3.8 | $CH_3$ | H | H | H | $C_6H_5$ | H | 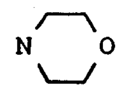 | Smp.: 131°C |
| 3.9 | $CH_3$ | H | H | H | 4-$CH_3$-$C_6H_4$ | H | NH-Propyl | |
| 3.10 | $CH_3$ | H | H | H | 2,4-$(CH_3)_2$-$C_6H_3$ | Br | NH Butyl | |
| 3.11 | $CH_3$ | H | H | H | 2,6-$(CH_3)_2$-$C_6H_3$ | Br | NHEt | |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 3.12 | $CH_3$ | H | H | H | 3-Et-$C_6H_4$ | H | 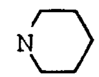 | |
| 3.13 | $CH_3$ | H | H | H | 3-Cl-$C_6H_4$ | H | NH Propyl | |
| 3.14 | $CH_3$ | H | H | H | 2,4-$Cl_2$-$C_6H_3$- | H | NH Me | |
| 3.15 | $CH_3$ | H | H | H | 4-$OCH_3$-$C_6H_4$- | H | $NMe_2$ | |
| 4.1 | $CH_3$ | H | H | H | Propyl | Br | NH Propyl | |
| 4.2 | $CH_3$ | H | H | H | Propyl | Br | N $Me_2$ | |

Tabelle A Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 4.3 | $CH_3$ | H | H | H | Propyl | Br | $NEt_2$ | |
| 4.4 | $CH_3$ | H | H | H | Propyl | Br | NH Et | |
| 4.5 | $CH_3$ | H | H | H | Propyl | Br | 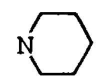 | |
| 4.6 | $CH_3$ | H | H | H | Propyl | Br | NH Butyl | |
| 4.7 | $CH_3$ | H | H | H | Propyl | Cl | NH Propyl | |
| 4.8 | $CH_3$ | H | H | H | Propyl | Cl | NH iso-Propyl | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 4.9 | $CH_3$ | H | H | H | Propyl | Cl | 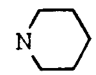 | |
| 4.10 | $CH_3$ | H | H | H | Propyl | Cl | $NCH_3CH_2C_6H_5$ | |
| 5.1 | $CH_3$ | H | H | H | $CH_2C_6H_5$ | H | $NEt_2$ | $^1H$-NMR ($CDCl_3$): d 8,16 t 7,66 m 7,29 d 7,19 s 5,94 s 4,19 t 3,48 s 2,7 t 1,12 [ppm] |
| 5.2 | $CH_3$ | H | H | H | $CH_2C_6H_5$ | H | 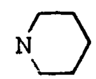 | $^1H$-NMR ($CDCl_3$): d 8,17 t 7,66 m 7,30 d 7,18 s 6,10 s 4,12 m 3,57 s 2,70 m 1,60 [ppm] |
| 5.3 | $CH_3$ | H | H | H | $CH_2C_6H_5$ | H | NH Propyl | $^1H$-NMR ($CDCl_3$): d 8,19 t 7,68 m 7,30 d 7,19 s 5,94 s 4,09 t 3,16 s 2,69 m 1,61 t 0,92 [ppm] |
| 5.4 | $CH_3$ | H | H | H | $CH_2C_6H_5$ | H | NH Et | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 5.5 | $CH_3$ | H | H | H | $CH_2C_6H_5$ | H | NH Butyl | |
| 5.6 | $CH_3$ | H | H | H | $CH_2C_6H_5$ | H | 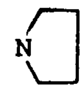 | |
| 5.7 | $CH_3$ | H | H | H | $CH_2C_6H_5$ | H | $NMe_2$ | |
| 5.8 | $CH_3$ | H | H | H | $CH_2C_6H_5$ | H | 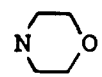 | Smp.: 161 - 163°C |
| 5.9 | $CH_3$ | H | H | H | $CH_2C_6H_5$ | H | NH Pentyl | |
| 5.10 | $CH_3$ | H | H | H | $CH_2C_6H_5$ | H | 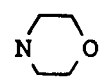 | $^1$-HNMR($CDCl_3$): d 8,17 t 7,68 s 7,23 d 7,19 s 6,09 s 4,16 m 3,84-3,52 s 2,70 [ppm] |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 5.11 | $CH_3$ | H | H | H | $CH_2C_6H_5$ | H | 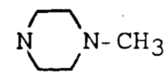 | $^1H$-NMR($CDCl_3$): d 8,18 t 7,68 s 7,29 d 7,19 s 6,12 s 4,18 t 3,64 s 2,70 t 2,43 s 2,30 [ppm] |
| 5.12 | $CH_3$ | H | H | H | $CH_2C_6H_5$ | H | $NHC_6H_4$-4-Cl | Smp.: 172 - 174°C |
| 6.1 | $CH_3$ | H | H | H | $CH_2OC_6H_5$ | H | $NHEt_2$ | $^1H$-NMR ($CDCl_3$): d 8,21 t 7,71 m 7,24 m 6,98 s 6,60 s 5,23 q 3,59 s 2,72 t 1,19 [ppm] |
| 6.2 | $CH_3$ | H | H | H | $CH_2OC_6H_5$ | H | 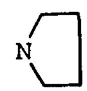 | Smp.: 122°C |
| 6.3 | $CH_3$ | H | H | H | $CH_2OC_6H_5$ | H | $NMe_2$ | Smp.: 134°C |
| 6.4 | $CH_3$ | H | H | H | $CH_2CH_2$-Cyclopentyl | H | 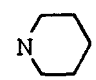 | $^1H$-NMR ($CDCl_3$): d 8,16 t 7,64 d 7,17 s 6,35 s 2,6 m 1,65 m 1,20 [ppm] |

Tabelle A Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 6.5 | $CH_3$ | H | H | H | $CH_2CH_2$-Cyclopentyl | H | NH Propyl | $^1H$-NMR ($CDCl_3$): d 8,15 t 7,62 d 7,16 s 6,16 t 3,26 s 2,63 m 1,71 t 0,97 [ppm] |
| 6.6 | $CH_3$ | H | H | H | $CH_2CH_2$-Cyclopentyl | H | NHMe | $^1H$-NMR ($CDCl_3$): d 8,19 t 7,69 d 1,9 s 6,19 q 5,60 d 2,94 s 2,66 m 1,69 [ppm] |
| 6.7 | $CH_3$ | H | H | H | $CH_2CH_2$-Cyclopentyl | H | $NCH_3CH_2C_6H_5$ | $^1H$-NMR ($CDCl_3$): d 8,14 t 7,64 m 7,26 d 7,16 s 6,31 s 4,89 s 3,13 s 2,70 m 1,62 [ppm] |
| 6.8 | $CH_3$ | H | H | H | $CH_2OC_6H_5$ | H | NH Propyl | Smp.: 152 - 153°C |
| 6.9 | $CH_3$ | H | H | H | $CH_2CH_2$Cyclopentyl | H | 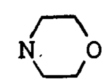 | Smp.: 98 - 100°C |
| 6.10 | $CH_3$ | H | H | H | $CH_2OC_6H_5$ | H | $NEt_2$ | Smp.: 142°C |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 6.11 | $CH_3$ | H | H | H | $CH_2OC_6H_5$ | H | 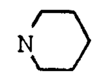 | Smp.: 122 - 123°C |
| 6.12 | $CH_3$ | H | H | H | $CH_2OC_6H_5$ | H | $N(CH_3)_2$ | Smp.: 134 - 136°C |
| 6.13 | $CH_3$ | H | H | H | $CH_2OC_6H_5$ | H | 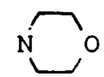 | Smp.: 125°C |
| 6.14 | $CH_3$ | H | H | H | $CH_2OC_6H_5$ | H | $NCH_3C_6H_5$ | Smp.: 109°C |
| 7.1 | $CH_3$ | H | H | H | $CH_3$ | Cl | $NEt_2$ | $^1H$-NMR ($CDCl_3$): d 8,11 t 7,66 d 7,18 q 3,69 s 2,70 s 2,66 t 1,33 [ppm] |
| 7.2 | $CH_3$ | H | H | H | $CH_3$ | Cl | $NMe_2$ | $^1H$-NMR ($CDCl_3$): d 8,16 t 7,68 d 7,19 q 3,26 s 2,70 s 2,64 [ppm] |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 7.3 | $CH_3$ | H | H | H | $CH_3$ | Cl | NH Propyl | Smp.: 106 - 107 |
| 7.4 | $CH_3$ | H | H | H | $CF_3$ | H | NH Propyl | |
| 7.5 | $CH_3$ | H | H | H | $CF_3$ | H | NH Butyl | |
| 7.6 | $CH_3$ | H | H | H | $MeOCH_2$ | $OCH_3$ | 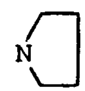 | |
| 7.7 | $CH_3$ | H | H | H | $MeOCH_2$ | $OCH_3$ | $NHC_3H_7$ | $^1H$-NMR ($CDCl_3$): d 8,14 t 7,64 d 7,16 s 4,56 s 3,81 s 3,46 s 2,64 [ppm] |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 7.8 | $CH_3$ | H | H | H | $MeOCH_2$ | $OCH_3$ | $NEt_2$ | $^1H$-NMR($CDCl_3$): d 8,08 t 7,64 d 7,16 s 4,59 q 3,67 s 3,49 s 2,66 t 1,24 [ppm] |
| 7.9 | $CH_3$ | H | H | H | $MeOCH_2$ | $OCH_3$ | $NHC_6H_5$ | |
| 7.10 | $CH_3$ | H | H | H | $H_3COCH_2$ | $OCH_3$ | $NHC_2H_5$ | Smp.: 102°C |
| 7.11 | $CH_3$ | H | H | H | $H_3COCH_2$ | $OCH_3$ | $NHCH_2C_6H_5$ | Smp.: 120 - 121°C |
| 7.12 | $CH_3$ | H | H | H | $H_3COCH_2$ | $OCH_3$ | 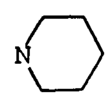 | Smp.: 129°C |
| 7.13 | $CH_3$ | H | H | H | $C_3H_7$ | Br | $NHC_3H_7$ | Smp.: 77°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 7.14 | $CH_3$ | H | H | H | $C_3H_7$ | Br | $N(C_2H_5)_2$ | Smp.: 95°C |
| 7.15 | $CH_3$ | H | H | H | $C_3H_7$ | Br | 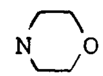 | Smp.: 89°C |
| 7.16 | $CH_3$ | H | H | H | $C_3H_7$ | Br | $N(CH_3)_2$ | Smp.: 103°C |
| 7.17 | $CH_3$ | H | H | H | $C_3H_7$ | Br | 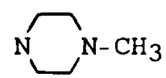 | |
| 7.18 | $CH_3$ | H | H | H | $CH_3$ | Cl | 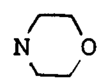 | Smp.: 72 - 74°C |
| 7.19 | $CH_3$ | H | H | H | $CH_3$ | Cl | 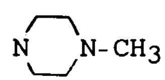 | Smp.: 80 - 83°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 7.20 | $CH_3$ | H | H | H | $CH_3$ | Cl | 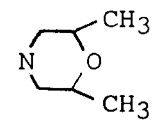 | Smp.: 95 - 97°C |
| 8.1 | $CH_3$ | H | H | H | | $-(CH_2)_4-$ | $NHCH_3$ | $^1$H-NMR ($CDCl_3$): d 8,21 t 7,66 d 7,17 s 3,14 m 2,91 s 2,66 m 2,35 m 1,84 [ppm] |
| 8.2 | $CH_3$ | H | H | H | | $-(CH_2)_4-$ | 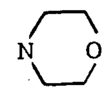 | $^1$H-NMR ($CDCl_3$): d 8,14 t 7,68 d 7,17 m 3,85 m 3,47 m 3,05 s 2,68 m 2,59 m 1,86 [ppm] |
| 8.3 | $CH_3$ | H | H | H | | $-(CH_2)_4-$ | 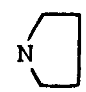 | $^1$H-NMR ($CDCl_3$): d 8,16 t 7,64 d 7,15 m 3,71 t 2,97 t 2,78 s 2,66 m 1,86 [ppm] |
| 8.4 | $CH_3$ | H | H | H | | $-(CH_2)_4-$ | NH Propyl | Smp.: 170 - 172°C |
| 8.5 | $CH_3$ | H | H | H | | $-(CH_2)_4-$ | $NEt_2$ | Smp.: 153 - 154°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 8.6 | $CH_3$ | H | H | H | $-(CH_2)_4-$ | | 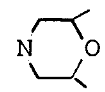 | Smp.: 141 - 144°C |
| 8.7 | $CH_3$ | H | H | H | $-(CH_2)_4-$ | | $NHCH_2C{\equiv}C\text{-}H$ | |
| 8.8 | $CH_3$ | H | H | H | $-(CH_2)_4-$ | | $NCH_3CH_2C_6H_5$ | |
| 9.1 | $CH_3$ | H | H | H | H | $OCH_3$ | NH Propyl | |
| 9.2 | $CH_3$ | H | H | H | H | $OCH_3$ | 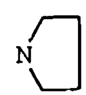 | |
| 9.3 | $CH_3$ | H | H | H | H | H | NH Butyl | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 9.4 | $CH_3$ | H | H | H | H | H | NH Propyl | |
| 9.5 | $CH_3$ | H | H | H | Cl | H | $NEt_2$ | $^1H$-NMR ($CDCl_3$): d 8,14 t 7,54 d 7,20 s 6,38 q 3,58 s 2,68 t 1,23 [ppm] |
| 9.6 | $CH_3$ | H | H | H | Cl | H | 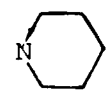 | |
| 9.7 | $CH_3$ | H | H | H | Cl | $OCH_3$ | NH Pentyl | |
| 9.8 | $CH_3$ | H | H | H | NH Propyl | H | NH Propyl | |
| 9.9 | $CH_3$ | H | H | H | NH Et | H | NH Propyl | |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 9.10 | $CH_3$ | H | H | H | $OC_4H_9$ | H | NH Et | |
| 9.11 | $CH_3$ | H | H | H | $OCH_3$ | H | $NEt_2$ | |
| 9.12 | $CH_3$ | H | H | H | SMe | H | NHMe | |
| 9.13 | $CH_3$ | H | H | H | $S-C_6H_4-4-Cl$ | $CH_3$ | NH Butyl | |
| 9.14 | $CH_3$ | H | H | H | H | Et | NH Propyl | |
| 9.15 | $CH_3$ | H | H | H | H | $CH_3$ | NH Propyl | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 9.16 | $CH_3$ | H | H | H | $NHC_3H_7$ | H | $NEt_2$ | Smp.: 79 - 81°C |
| 9.17 | $CH_3$ | H | H | H | Cl | H | $NEt_2$ | $^1$H-NMR($CDCl_3$): d 8,14 t 7,68 d 7,21 s 6,37 q 3,56 s 2,67 t 1,20 [ppm] |
| 9.18 | $CH_3$ | H | H | H | Cl | H | 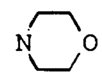 | Smp.: 159°C |
| 9.19 | $CH_3$ | H | H | H | $OC_2H_5$ | H | $NHC_3H_7$ | Smp.: 135°C |
| 10.1 | $C_6H_5$ | H | H | H | $CH_3$ | H | $NMe_2$ | $^1$H-NMR ($CDCl_3$): dd 8,36 s 6,30 s 3,22 s 2,51 [ppm] |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 10.2 | $C_6H_5$ | H | H | H | $CH_3$ | H | $NEt_2$ | $^1H$-NMR ($CDCl_3$): dd 8,32 s 6,26 q 3,62 s 2,51 t 1,23 [ppm] |
| 10.3 | $C_6H_5$ | H | H | H | $CH_3$ | H | NH Propyl | $^1H$-NMR ($CDCl_3$): dd 8,33 s 6,19 t 3,24 s 2,47 m 1,65 t 0,98 [ppm] |
| 10.4 | $C_6H_5$ | H | H | H | $CH_3$ | H | NH iso-Propyl | $^1H$-NMR ($CDCl_3$): dd 8,39 s 6,17 sept 3,92 s 2,50 d 1,28 [ppm] |
| 10.5 | $C_6H_5$ | H | H | H | $CH_3$ | $CH_3$ | NH-(3,5-$Cl_2$-$C_6H_3$) | |
| 11.1 | $C_6H_5$ | H | H | H | $C_3H_7$ | H | $NEt_2$ | $^1H$-NMR ($CDCl_3$): dd 8,28 s 6,21 q 3,61 t 2,73 m 1,80 t 1,24 t 1,02 [ppm] |
| 11.2 | $C_6H_5$ | H | H | H | $C_3H_7$ | H | NH Propyl | $^1H$-NMR ($CDCl_3$): dd 8,31 s 6,15 t 5,28 m 3,27 t 2,70 m 1,72 t 1,01 t 1,03 [ppm] |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 11.3 | $C_6H_5$ | H | H | H | $C_3H_7$ | H | $NMe_2$ | $^1H$-NMR ($CDCl_3$): dd 8,33 s 6,26 s 3,20 t 2,76 m 1,82 t 1,00 [ppm] |
| 11.4 | $C_6H_5$ | H | H | H | $C_3H_7$ | H | $NH-CH_2-CH=CH_2$ | |
| 11.5 | $C_6H_5$ | H | H | H | $C_3H_7$ | H | $NHCH_2-CH=CH-CH_3$ E-Isomeres | |
| 11.6 | $C_6H_5$ | H | H | H | $C_3H_7$ | $CH_3$ | $NHCH_2-CH=CH-CH_3$ Z-Isomeres | |
| 20.1 | H | H | H | H | $C_6H_5$ | H | $NEt_2$ | Smpkt.: 155 - 156°C |
| 20.2 | H | H | H | H | $C_6H_5$ | H | 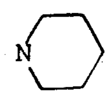 | $^1H$-NMR ($CDCl_3$): d 8,81 d 8,49 m 8,10 t 7,81 m 7,41 s 6,73 q 3,69 t 1,26 [ppm] |

Tabelle A Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 20.3 | H | H | H | H | $C_6H_5$ | H | NH Propyl | Smp.: 118 - 120°C |
| 20.4 | H | H | H | H | $C_6H_5$ | H | NH iso-Propyl | |
| 20.5 | H | H | H | H | $C_6H_5$ | H | NHMe | |
| 20.6 | H | H | H | H | $C_6H_5$ | H | 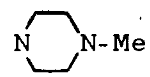 | |
| 20.7 | H | H | H | H | $C_6H_5$ | H | $NMe_2$ | |
| 20.8 | H | H | H | H | $C_6H_5$ | H | 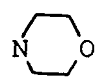 | Smp.: 189 - 190°C |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 20.9 | H | H | H | H | $CH_3$ | H | $NHC_3H_7$ | Smp.: 146°C |
| 20.10 | H | H | H | H | $CH_3$ | H | $NHCH_2CH(CH_3)_2$ | Smp.: 123°C |
| 20.11 | H | H | H | H | $CH_3$ | H | 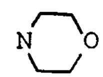 | Smp.: 82°C |
| 20.12 | H | H | H | H | $CH_3$ | H | $NHCH(CH_3)_2$ | Smp.: 115 -117°C |
| 30.1 | 4-Cl-$C_6H_4$-$OCH_2$ | H | H | H | $CH_3$ | H | $NMe_2$ | $^1$H-NMR ($CDCl_3$): d 8,34 t 7,85 d 7,56 d 7,23 d 6,92 s 6,32 |
| 30.2 | 4-Cl-$C_6H_4$-$OCH_2$ | H | H | H | $CH_3$ | H | $NEt_2$ | $^1$H-NMR ($CDCl_3$): d 8,30 t 7,81 d 7,53 d 7,22 d 6,92 s 6,25 s 5,39 q 3,62 s 2,52 t 1,23 [ppm] |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 30.3 | 4-Cl-$C_6H_4$-$OCH_2$ | H | H | H | $CH_3$ | H | NH Propyl | |
| 30.4 | $C_6H_5$-$OCH_2$ | H | H | H | $CH_3$ | H | NHEt | |
| 30.5 | 2,6-$(Me)_2$-$C_6H_3$ | H | H | H | $CH_3$ | H | 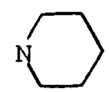 | |
| 31.1 | 4-Cl-$C_6H_4$-$OCH_2$ | H | H | H | Propyl | H | $NEt_2$ | $^1H$-NMR ($CDCl_3$): d 8,29 t 7,81 d 7,54 d 7,24 d 6,93 s 6,24 s 5,36 q 3,6 t 2,74 m 1,81 t 1,25 t 1,02 [ppm] |
| 31.2 | 4-Cl-$C_6H_4$-$OCH_2$ | H | H | H | Propyl | H | $NMe_2$ | $^1H$-NMR ($CDCl_3$): d 8,31 t 7,81 d 7,54 d 7,23 d 6,93 s 6,30 s 5,34 s 3,19 t 2,74 m 1,83 t 1,01 [ppm] |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 31.3 | 4-Cl-$C_6H_4$-$OCH_2$ | H | H | H | Propyl | H | NH Propyl | $^1$H-NMR ($CDCl_3$): d 8,31 t 7,82 d 7,55 d 7,24 d 6,91 d 6,18 s 5,34 m 3,26 t 2,71 m 1,70 t 1,01 [ppm] Smp.: 139 - 140°C |
| 31.4 | 4-Cl-$C_6H_4$-$OCH_2$ | H | H | H | Propyl | H | $NCH_3C_6H_5$ | |
| 40.1 | 4-Cl-$C_6H_4$-O-$C_6H_4$-$OCH_2$ | H | H | H | $CH_3$ | H | $NEt_2$ | $^1$H-NMR (DMSO-$d_6$): d 8,21 t 7,92 d 7,58 d 7,37 dd 7,09 d 6,94 s 6,51 s 5,28 q 3,58 s 2,35 t 1,14 [ppm] |
| 41.1 | 4-Cl-$C_6H_4$-O-$C_6H_4$-$OCH_2$ | H | H | H | Propyl | H | $NEt_2$ | $^1$H-NMR (DMSO-$d_6$): d 8,21 t 7,94 d 7,60 d 7,39 dd 7,09 d 6,94 s 6,50 s 5,23 q 3,59 t 2,63 m 1,71 t 1,15 t 0,96 [ppm] |
| 50.1 | $C_6H_5CH_2$ | H | H | H | Propyl | H | $NHC_6H_5$ | Smp.: 116°C |
| 50.2 | $C_6H_5CH_2$ | H | H | H | Propyl | H | $NCH_3C_6H_5$ | |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 50.3 | $C_6H_5CH_2$ | H | H | H | Propyl | H | NH Propyl | |
| 50.4 | $C_6H_5CH_2$ | H | H | H | Propyl | H | NH Pentyl | |
| 50.5 | $C_6H_5CH_2$ | H | H | H | Propyl | H | 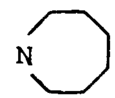 | |
| 50.6 | $C_6H_5CH_2$ | H | H | H | iso-Propyl | H | NH Propyl | |
| 50.7 | $C_6H_5CH_2$ | H | H | H | iso-Propyl | H | $NEt_2$ | |
| 50.8 | $C_6H_5CH_2$ | H | H | H | iso-Propyl | H | 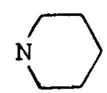 | |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 50.9 | $C_6H_5CH_2$ | H | H | H | iso-Propyl | H | 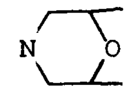 | |
| 50.10 | $C_6H_5CH_2$ | H | H | H | $C_3H_7$ | H | $N(C_2H_5)_2$ | Smp.: 98°C |
| 50.11 | $C_6H_5CH_2$ | H | H | H | $C_3H_7$ | H | $NHC_3H_7$ | Smp.: 127°C |
| 50.12 | $C_6H_5CH_2$ | H | H | H | $C_3H_7$ | H | $N(CH_3)_2$ | Smp.: 154°C |
| 50.13 | $C_6H_5CH_2$ | H | H | H | $C_3H_7$ | H | 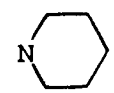 | Smp.: 115 - 117°C |
| 50.14 | $C_6H_5CH_2$ | H | H | H | $C_3H_7$ | H | 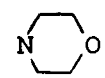 | Smp.: 162 - 163°C |
| 50.15 | $C_6H_5CH_2$ | H | H | H | $C_3H_7$ | H | 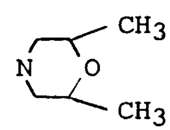 | Smp.: 164°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 50.16 | $C_6H_5CH_2$ | H | H | H | $C_3H_7$ | H | 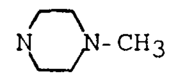 | Smp.: 134°C |
| 50.17 | $C_6H_5CH_2$ | H | H | H | $C_3H_7$ | H | 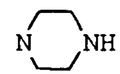 | Smp.: 170°C |
| 51.1 | $C_6H_5CH_2$ | H | H | H | $C_6H_5$ | H | $NHC_3H_7$ | Smp.: 147 - 148°C |
| 51.2 | $C_6H_5CH_2$ | H | H | H | $C_6H_5$ | H | $N(C_2H_5)_2$ | Smp.: 119°C |
| 51.3 | $C_6H_5CH_2$ | H | H | H | $C_6H_5$ | H | 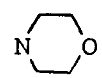 | Smp.: 99 - 101°C |
| 52.1 | $C_6H_5CH_2$ | H | H | H | $C_5H_9$ | H | 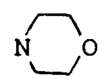 | Smp.: 139°C |
| 52.2 | $C_6H_5CH_2$ | H | H | H | $C_5H_9$ | H | $NHC_3H_7$ | Smp.: 147°C |
| 52.3 | $C_6H_5CH_2$ | H | H | H | $C_5H_9$ | H | $NHCH_2CH=CH_2$ | Smp.: 121-123°C |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 52.4 | $C_6H_5CH_2$ | H | H | H | $C_5H_9$ | H | $NHC_5H_9$ | Smp.: 133°C |
| 52.5 | $C_6H_5CH_2$ | H | H | H | $C_5H_9$ | H | 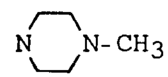 | $^1H$-NMR($CDCl_3$): d 8,16 t 7,64 m 7,37-7,18 d 8,01 s 6,38 s 4,35 t 3,78 m 2,90-2,74 t 2,52 m 1,91-1,60 m 1,50-1,19 m 1,02-0,76 [ppm] |
| 53.1 | $C_6H_5CH_2$ | H | H | H | $C_6H_5CH_2$ | H | $NHC_3H_7$ | Smp.: 149°C |
| 53.2 | $C_6H_5CH_2$ | H | H | H | $C_6H_5CH_2$ | H | $NHCHCH_3C_2H_5$ | Smp.: 162°C |
| 53.3 | $C_6H_5CH_2$ | H | H | H | $C_6H_5CH_2$ | H | 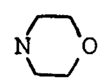 | Smp.: 114 - 116°C |
| 53.4 | $C_6H_5CH_2$ | H | H | H | $C_6H_5CH_2$ | H | $NHCH_2CH=CH_2$ | Smp.: 130 - 133°C |
| 53.5 | $C_6H_5CH_2$ | H | H | H | $C_6H_5CH_2$ | H | $NHCH_2C_6H_5$ | Smp.: 145°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 71.1 | $H_3COCH_2$ | H | H | H | Propyl | H | 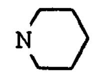 | $^1H$-NMR ($CDCl_3$): d 8,24 t 7,80 d 7,49 s 6,35 s 4,76 m 3,69 t 2,71 m 1,65 t 0,97 [ppm] |
| 71.2 | $H_3COCH_2$ | H | H | H | Propyl | H | NH Propyl | |
| 71.3 | $H_3COCH_2$ | H | H | H | iso-Propyl | H | NH Propyl | |
| 71.4 | $CH_3OCH_2$ | H | H | H | Propyl | H | $NCH_2C_6H_5$ | |
| 71.5 | $CH_3OCH_2$ | H | H | H | Propyl | H | 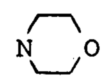 | Smp.: 116 -117°C |
| 72.1 | $H_3COCH_2$ | H | H | H | $C_6H_5$ | H | NH Propyl | $^1H$-NMR ($CDCl_3$): d 8,50 m 8,13 t 7,85 m 7,49 s 6,71 s 4,79 s 3,49 m 3,36 m 1,72 t 1,04 [ppm] |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 72.2 | $H_3COCH_2$ | H | H | H | $C_6H_5$ | H | $NEt_2$ | $^1H$-NMR ($CDCl_3$): d 8,35 m 8,11 t 7,82 m 7,46 s 6,74 s 4,78 q 3,68 s 3,49 t 1,24 [ppm] |
| 72.3 | $H_3COCH_2$ | H | H | H | $C_6H_5$ | H | 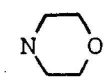 | Smp.: 147 - 148°C |
| 80.1 | $C_3H_7$ | H | H | H | $C_3H_7$ | H | NH Propyl | |
| 80.2 | $C_3H_7$ | H | H | H | $C_3H_7$ | H | 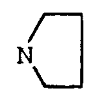 | |
| 80.3 | $C_3H_7$ | H | H | H | $C_3H_7$ | H | $NMe_2$ | |
| 80.4 | $C_3H_7$ | H | H | H | $CH(CH_3)_2$ | H | NH Butyl | |
| 80.5 | $C_3H_7$ | H | H | H | $CH(CH_3)_2$ | H | NH Propyl | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 80.6 | $C_3H_7$ | H | H | H | $CH(CH_3)_2$ | H | NHEt | |
| 80.7 | $C_3H_7$ | H | H | H | $C_6H_5$ | H | NH Butyl | |
| 90.1 | $CH_3$ | H | $CH_3$ | H | Propyl | H | NH Propyl | |
| 90.2 | $CH_3$ | H | $CH_3$ | H | Propyl | H | NHEt | |
| 90.3 | $CH_3$ | H | $CH_3$ | H | Propyl | H | $NMe_2$ | |
| 90.4 | $CH_3$ | H | $CH_3$ | H | iso-Propyl | H | 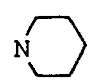 | |

Tabelle A Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 100.1 | H | H | Et | H | Propyl | H | NH Propyl | |
| 100.2 | H | H | Et | H | Propyl | H | 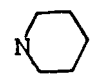 | |
| 100.3 | H | H | Et | H | Propyl | H | NH-iso-Propyl | |
| 100.4 | H | H | Et | H | $C_6H_5$ | H | NHEt | |
| 100.5 | H | H | Et | H | $C_6H_5$ | H | $NEt_2$ | |
| 100.6 | H | H | Et | H | $C_6H_5$ | H | $NMe_2$ | |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 100.7 | H | H | Et | H | iso-Propyl | H | NH Propyl | |
| 100.8 | H | H | Et | H | iso-Propyl | H | $NMe_2$ | |
| 101.1 | $CH_3$ | $CH_3$ | H | H | Propyl | H | NH Propyl | Smp.: 108°C |
| 101.2 | $CH_3$ | $CH_3$ | H | H | Propyl | H | NHMe | Smp.: 127 - 128°C |
| 101.3 | $CH_3$ | $CH_3$ | H | H | $C_3H_7$ | H | 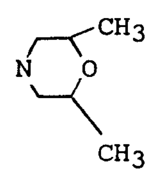 | Smp.: 133°C |
| 101.4 | $CH_3$ | $CH_3$ | H | H | $C_3H_7$ | H | 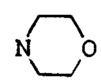 | Smp: 125°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 101.5 | $CH_3$ | $CH_3$ | H | H | $C_3H_7$ | H | 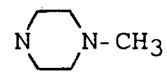 | Smp: 87°C |
| 101.6 | $CH_3$ | $CH_3$ | H | H | $C_6H_5CH_2$ | | 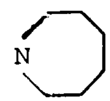 | Smp.: 56°C |
| 101.7 | $CH_3$ | $CH_3$ | H | H | $C_6H_5CH_2$ | | $NHC_5H_{11}$ | Smp.: 111°C |
| 101.8 | $CH_3$ | $CH_3$ | H | H | $C_6H_5CH_2$ | | 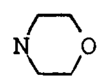 | Smp.: 127°C |
| 101.9 | $CH_3$ | $CH_3$ | H | H | $-(CH_2)_4-$ | | $NHC_3H_7$ | Smp.: 80°C |
| 101.10 | $CH_3$ | $CH_3$ | H | H | $-(CH_2)_4-$ | | 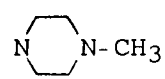 | Smp.: 155°C |
| 101.11 | $CH_3$ | $CH_3$ | H | H | $-(CH_2)_4-$ | | $NHC_5H_{11}$ | Smp.: 86 - 88°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 102.1 | Propyl | H | H | H | H | $C_6H_5$ | $NEt_2$ | Smp.: 96°C |
| 102.2 | Propyl | H | H | H | H | $C_6H_5$ | NHEt | Smp.: 112 -113°C |
| 102.3 | $C_3H_7$ | H | H | H | $C_6H_5CH_2$ | H | $NHC_3H_7$ | Smp.: 139°C |
| 102.4 | $C_3H_7$ | H | H | H | $C_6H_5CH_2$ | H | 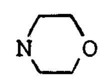 | Smp.: 185°C |
| 102.5 | $C_3H_7$ | H | H | H | $C_6H_5CH_2$ | H | 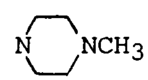 | Smp.: 135 - 136°C |
| 102.6 | $C_3H_7$ | H | H | H | $C_6H_5CH_2$ | H | $NHC_5H_{11}$ | Smp.: 112°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 102.7 | $C_3H_7$ | H | H | H | $C_6H_5CH_2$ | H | $NHCH_2C_6H_5$ | Smp.: 156 - 159°C |
| 102.8 | $C_3H_7$ | H | H | H | $(CH_2)_2$-Cyclopentyl | H | $NHC_3H_7$ | Smp.: 112 - 114°C |
| 102.9 | $C_3H_7$ | H | H | H | $(CH_2)_2$-Cyclopentyl | H | 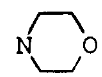 | Smp.: 174°C |
| 102.10 | $C_3H_7$ | H | H | H | $(CH_2)_2$-Cyclopentyl | H | 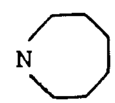 | Smp.: 170°C |
| 102.11 | $C_3H_7$ | H | H | H | $(CH_2)_2$-Cyclopentyl | H | $NHCH_2CH{=}CH_2$ | Smp.: 141 - 143°C |
| 102.12 | $C_3H_7$ | H | H | H | $(CH_2)_2$-Cyclopentyl | H | 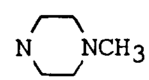 | Smp.: 158 - 160°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 102.13 | $C_3H_7$ | H | H | H | $CH_3OCH_2$ | $OCH_3$ | $NHC_3H_7$ | Smp.: 87 - 89°C |
| 102.14 | $C_3H_7$ | H | H | H | $CH_3OCH_2$ | $OCH_3$ | 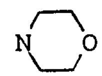 | Smp.: 135°C |
| 102.15 | $C_3H_7$ | H | H | H | $CH_3OCH_2$ | $OCH_3$ | 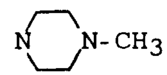 | Smp.: 121 - 122°C |
| 102.16 | $C_3H_7$ | H | H | H | $CH_3$ | Cl | $NHC_3H_7$ | Smp.: 99°C |
| 102.17 | $C_3H_7$ | H | H | H | $CH_3$ | Cl | 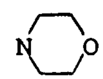 | Smp.: 161°C |
| 102.18 | $C_3H_7$ | H | H | H | $CH_3$ | Cl | $NHCH_2CH=CH_2$ | Smp.: 128°C |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 102.19 | $C_3H_7$ | H | H | H | $CH_3$ | Cl | $NHC_5H_{11}$ | Smp.: 112°C |
| 102.20 | $C_3H_7$ | H | H | H | $C_3H_7$ | N | 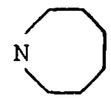 | Smp.: 61 - 63°C |
| 102.21 | $C_3H_7$ | H | H | H | $C_3H_7$ | H | $NHC_5H_{11}$ | Smp.: 75°C |
| 102.22 | $C_3H_7$ | H | H | H | $C_3H_7$ | H | $NHC_3H_7$ | Smp.: 86 - 89°C |
| 102.23 | $C_3H_7$ | H | H | H | $C_3H_7$ | H | 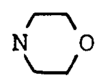 | Smp.: 75°C |
| 102.24 | $C_3H_7$ | H | H | H | $C_3H_7$ | H | 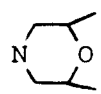 | Smp.: 66 - 68°C |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 102.25 | $C_3H_7$ | H | H | H | $C_3H_7$ | N | $NHCH(CH_3)_2$ | Smp.: 98 - 100°C |
| 102.26 | $C_3H_7$ | H | H | H | $C_3H_7$ | N | 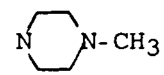 | Smp.: 85°C |
| 102.27 | $C_3H_7$ | H | H | H | $C_3H_7$ | H | 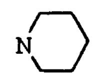 | Smp.: 109 - 110°C |
| 102.28 | $C_3H_7$ | H | H | H | $C_3H_7$ | H | $NHCH_2C_6H_5$ | Smp.: 94°C |
| 102.29 | $C_3H_7$ | H | H | H | $-(CH_2)_4-$ | | $NHC_3H_7$ | Smp.: 135 - 137°C |
| 102.30 | $C_3H_7$ | H | H | H | $-(CH_2)_4-$ | | $NHC_5H_{11}$ | Smp.: 128 - 130°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 102.31 | $C_3H_7$ | H | H | H | $-(CH_2)_4-$ | | 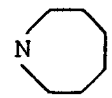 | Smp.: 125 - 126°C |
| 102.32 | $C_3H_7$ | H | H | H | $-(CH_2)_4-$ | | 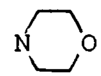 | Smp.: 111 - 113°C |
| 102.33 | $C_3H_7$ | H | H | H | $OC_2H_5$ | H | $NHC_5H_{11}$ | |
| 110.1 | H | H | $C_6H_5$ | H | Propyl | H | NHMe | |
| 110.2 | H | H | $C_6H_5$ | H | Propyl | H | $NMe_2$ | |
| 110.3 | H | H | $C_6H_5$ | H | Propyl | H | NH Propyl | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 110.4 | H | H | $C_6H_5$ | H | Propyl | H | NH-Cyclohexyl | |
| 110.5 | H | H | $C_6H_5$ | H | Propyl | H | 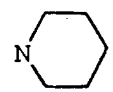 | |
| 110.6 | H | H | $C_6H_5$ | H | iso-Propyl | H | NH Propyl | |
| 110.7 | H | H | $C_6H_5$ | H | iso-Propyl | H | NHEt | |
| 110.8 | H | H | $C_6H_5$ | H | iso-Propyl | H | NH-Cyclopropyl | |
| 110.9 | H | H | $C_6H_5$ | H | iso-Propyl | H | 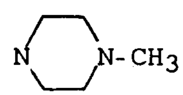 | |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 110.10 | H | H | $C_6H_5$ | H | Phenyl | H | NH Proypl | |
| 110.11 | H | H | $C_6H_5$ | H | Phenyl | H | NHEt | |
| 110.12 | H | H | $C_6H_5$ | H | Phenyl | H | NH iso-Propyl | |
| 110.13 | H | H | $C_6H_5$ | H | $C_3H_7$ | H | $NHC_5H_{11}$ | Smp.: 107°C |
| 110.14 | H | H | $C_6H_5$ | H | $C_3H_7$ | H | 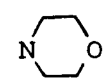 | Smp.: 117 - 119°C |
| 110.15 | H | H | $C_6H_5$ | H | $C_3H_7$ | H | 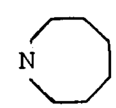 | Smp.: 125 - 126°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 110.16 | H | H | $C_6H_5$ | H | $C_3H_7$ | H | $NHCH_2C_6H_5$ | Smp.: 132°C |
| 110.17 | H | H | $C_6H_5$ | H | $C_6H_5CH_2$ | H | $NHC_3H_7$ | Smp.: 139°C |
| 110.18 | H | H | $C_6H_5$ | H | $C_6H_5CH_2$ | H | 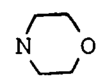 | Smp.: 140°C |
| 110.19 | H | H | $C_6H_5$ | H | $C_6H_5CH_2$ | H | 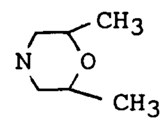 | Smp.: 148 - 150°C |
| 110.20 | H | H | $C_6H_5$ | H | $C_6H_5CH_2$ | H | 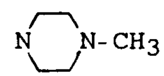 | $^1H$-NMR($CDCl_3$): d 8,86 s(br) 8,64 m 7,78-7,26 s 6,18 s 4,19 t 3,71 t 2,43 s 2,29 [ppm] |
| 120.1 | $CH_3$ | H | $CH_3$ | H | $C_3H_7$ | H | 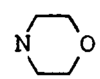 | Smp.: 155°C |

**Tabelle A** Fortsetzung

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 120.2 | $CH_3$ | H | $CH_3$ | H | $C_3H_7$ | H | 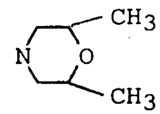 | Smp.: 169 - 170°C |
| 120.3 | $CH_3$ | H | $CH_3$ | H | $C_3H_7$ | H | 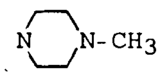 | $^1H$-NMR($CDCl_3$): s(br) 7,96 s(br) 7,03 s 6,38 t 3,71 t 2,74 s 2,64 t 2,53 s 2,36 dq 1,79 t 0,99 [ppm] |
| 120.4 | $CH_3$ | H | $CH_3$ | H | $C_3H_7$ | H | $NHC_3H_7$ | Smp.: 86 - 88°C |
| 120.5 | $CH_3$ | H | $CH_3$ | H | $C_3H_7$ | H | 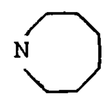 | Smp.: 112°C |
| 120.6 | $CH_3$ | H | $CH_3$ | H | $C_3H_7$ | H | 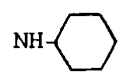 | Smp.: 131°C |
| 120.7 | $CH_3$ | H | $CH_3$ | H | $C_6H_5CH_2$ | H | 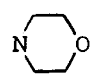 | Smp.: 148°C |

**Tabelle A Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 120.8 | $CH_3$ | H | $CH_3$ | H | $C_6H_5CH_2$ | H | 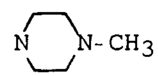 | $^1$H-NMR($CDCl_3$): s(br) 7,99 s(br) 7,05 s 6,11 s 4,18 t 3,65 s 2,63 t 2,45 s 2,38 s 2,31 [ppm] |

**Tabelle B**

(Säureadditionssalze)

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 200.1 | $CH_3$ | H | H | H | $C_6H_5$ | H | NH Propyl | semikristallin, farblos |
| 200.2 | $CH_3$ | H | H | H | $C_3H_7$ | H | NH Et | |
| 200.3 | $CH_3$ | H | H | H | $C_3H_7$ | Br | NH Propyl | |
| 200.4 | $C_6H_5$ | H | H | H | $CH_3$ | H | NH Butyl | |

**Tabelle B Fortsetzung**

| Nr. | $R^1$ | $R^2$ | $R^3$ | $R^4$ | $R^5$ | $R^6$ | $NR^7R^8$ | physikalische Eigenschaften |
|---|---|---|---|---|---|---|---|---|
| 200.5 | $C_3H_7$ | H | H | H | $CH_2CH_2$-Cyclopentyl | H | NHEt | |

## C. Biologische Beispiele

Filterpapierscheibchen von 6 mm Durchmesser werden mit je 20 µl der in Tabelle 1 angegebenen Wirkstoffe gleichmäßig benetzt und auf ein, je nach Pilzart, unterschiedliches Agar-Medium aufgelegt. Dem

Agar werden zuvor in noch flüssigem Zustand je Petrischale 0,5 ml Suspensionskultur des Testorganismus (im vorliegenden Fall Botrytis cinerea, BCM- und Iprodion resistenter Stamm, ca. $10^5$ - $10^6$ Konidien) zugegeben und die so behandelten Agarplatten anschließend bei ca. 22 °C bebrütet. Nach 3 - 4 tägiger Inkubation wird die Inhibitionszone als Maß der Pilzhemmung gemessen und in mm angegeben.

Tabelle 1

| Fungizide Wirkung gegenüber Botrytis cinerea - BCM- und Iprodion-resistenter Stamm. | |
|---|---|
| Verbindung gemäß Beispiel | Hemmzonen in mm Durchmesser bei 1000 ppm Wirkstoff und 20 µl pro Filterscheibchen |
| 1.1 | 28 |
| 1.2 | 26 |
| 1.3 | 30 |
| 1.4 | 24 |
| 2.7 | 32 |
| 2.1 | 12 |
| 2.38 | 12 |
| 2.2 | 44 |
| 7.1 | 14 |
| 7.3 | 40 |
| 10.2 | 14 |
| Verbindungen gemäß Beispiel | Hemmzonen in mm Durchmesser bei 100 ppm Wirkstoff und 20 µl pro Filterscheibchen |
| 11.1 | 22 |
| 11.2 | 20 |
| 11.3 | 22 |
| 31.4 | 16 |
| unbehandelte Kontrolle | 0 |

**Beispiel 2**

Filterpapierscheibchen von 6 mm Durchmesser werden mit je 20 µl der in Tabelle 2 angegebenen Wirkstoffe gleichmäßig benetzt und auf ein, je nach Pilzart, unterschiedliches Agar-Medium aufgelegt. Dem Agar werden zuvor in noch flüssigem Zustand je Petrischale 0,5 ml Suspensionskultur des Testorganismus (im vorliegenden Fall Alternaria mali) zugegeben und die so behandelten Agarplatten anschließend bei ca. 22 °C bebrütet. Nach 3 - 4 tägiger Inkubation wird die Inhibitionszone als Maß der Pilzhemmung gemessen und in mm angegeben.

Tabelle 2

| Fungizide Wirkung gegenüber Alternaria mali. | |
|---|---|
| Verbindungen gemäß Beispiel | Hemmzonen in mm Durchmesser bei 1000 ppm Wirkstoff und 20 µl pro Filterscheibchen |
| 2.2 | 20 |
| 7.1 | 36 |
| 7.3 | 36 |
| 10.1 | 14 |
| 10.2 | 14 |
| 10.4 | 26 |
| 11.1 | 30 |
| 11.2 | 30 |
| 11.3 | 30 |
| 31.3 | 16 |
| unbehandelte Kontrolle | 0 |

**Beispiel 3**

Filterpapierscheibchen von 6 mm Durchmesser werden mit je 20 µl der in Tabelle 3 angegebenen Wirkstoffe gleichmäßig benetzt und auf ein, je nach Pilzart, unterschiedliches Agar-Medium aufgelegt. Dem Agar werden zuvor in noch flüssigem Zustand je Petrischale 0,5 ml Suspensionskultur des Testorganismus (im vorliegenden Fall Sclerotinia sclerotiorum, Hyphenstücke des Pilzes) zugegeben und die so behandelten Agarplatten anschließend bei ca. 22 °C bebrütet. Nach 3 - 4 tägiger Inkubation wird die Inhibitionszone als Maß der Pilzhemmung gemessen und in mm angegeben.

Tabelle 3

| Fungizide Wirkung gegenüber Sclerotinia sclerotiorum | |
|---|---|
| Verbindungen gemäß Beispiel | Hemmzonen in mm Durchmesser bei 1000 ppm Wirkstoff und 20 µl pro Filterscheibchen |
| 2.2 | 14 |
| 7.1 | 40 |
| 7.3 | 50 |
| 10.2 | 14 |
| 10.4 | 20 |
| 30.1 | 12 |
| 31.2 | 20 |
| unbehandelte Kontrolle | 0 |

**Beispiel 4**

Gerstenpflanzen wurden im 2-Blattstadium mit Konidien des Gerstenmehltaus (Erysiphe graminis hordei) stark inokuliert und in einem Gewächshaus bei 20 °C und einer relativen Luftfeuchte von ca. 50 % weiterkultiviert. 1 Tag nach Inokulation wurden die Pflanzen mit den in Tabelle 4 aufgeführten Verbindungen in den angegebenen Wirkstoffkonzentrationen gleichmäßig benetzt. Nach einer Inkubationszeit von 7 - 9 Tagen wurden die Pflanzen auf Befall mit Gerstenmehltau untersucht. Der Wirkungsgrad der Prüfsubstanzen wurde prozentual zur unbehandelten, infizierten Kontrolle bonitiert und ist in Tabelle 4 wiedergegeben.

Tabelle 4

| Verbindungen gemäß Beispiel | Wirkungsgrad in % bei mg Wirkstoff/Liter Spritzbrühe 500 |
|---|---|
| 9.17 | 100 |
| 2.49 | 100 |
| 7.8 | 100 |
| 7.12 | 90 |
| 8.2 | 90 |
| 8.5 | 100 |
| 7.14 | 100 |
| 7.15 | 100 |
| 7.16 | 100 |
| 2.8 | 90 |
| 2.11 | 100 |
| 101.1 | 100 |
| 6.9 | 90 |
| 102.11 | 100 |
| 102.21 | 100 |
| 102.16 | 100 |
| 102.17 | 100 |
| 102.33 | 100 |
| unbehandelte, infizierte Pflanzen | 0 |

**Beispiel 5**

Ca. 14 Tage alte Ackerbohnen der Sorten "Harz Freya" oder "Frank's Ackerperle" wurden mit wässrigen Suspensionen der beanspruchten Verbindungen tropfnaß behandelt.

Nach Antrocknen des Spritzbelages wurden die Pflanzen mit einer Sporensuspension (1,5 Mio Sporen/ml) von Botrytis cinerea inokuliert. Die Pflanzen wurden in einer Klimakammer bei 20 - 22 ° C und ca. 99 % rel. Luftfeuchte weiterkultiviert. Die Infektion der Pflanzen äußert sich in der Bildung schwarzer Flecken auf Blättern und Stengeln. Die Auswertung der Versuche erfolgte ca. 1 Woche nach Inokulation.

Der Wirkungsgrad der Prüfsubstanzen wurde prozentual zur unbehandelten, infizierten Kontrolle bonitiert und ist in Tabelle 5 wiedergegeben.

Tabelle 5

| Verbindungen gemäß Beispiel | Wirkungsgrad in % bei mg Wirkstoff/ Liter Spritzbrühe 500 |
|---|---|
| 2.15 | 100 |
| 5.8 | 90 |
| 2.33 | 90 |
| 2.9 | 100 |
| 5.11 | 100 |
| 72.3 | 100 |
| 101.1 | 100 |
| 110.20 | 90 |
| 101.5 | 100 |
| 101.10 | 100 |
| 101.11 | 100 |
| 120.3 | 100 |
| 5.12 | 90 |
| 6.9 | 90 |
| 102.7 | 90 |
| 102.11 | 100 |
| 102.21 | 90 |
| 102.22 | 90 |
| 102.8 | 100 |
| 102.3 | 100 |
| 102.17 | 100 |
| 102.4 | 100 |
| 102.5 | 100 |
| 102.13 | 90 |
| 102.26 | 90 |
| 102.15 | 100 |
| 102.14 | 100 |
| 102.32 | 100 |

Fortsetzung Tabelle 5

| Verbindungen gemäß Beispiel | Wirkungsgrad in % bei mg Wirkstoff/ Liter Spritzbrühe 500 |
|---|---|
| 6.13 | 100 |
| 6.8 | 100 |
| 9.16 | 90 |
| 9.18 | 90 |
| 2.34 | 90 |
| 2.41 | 100 |
| 2.40 | 100 |
| 2.42 | 90 |
| 2.45 | 90 |
| 2.48 | 90 |
| 7.18 | 100 |
| 2.49 | 100 |
| 2.51 | 90 |
| 7.19 | 100 |
| 7.8 | 100 |
| 52.4 | 90 |
| 52.3 | 100 |
| 8.1 | 90 |
| 8.2 | 100 |
| 8.5 | 90 |
| 7.13 | 90 |
| 7.14 | 90 |
| 7.15 | 90 |
| 2.8 | 90 |
| 3.7 | 90 |
| 2.11 | 100 |
| 2.13 | 100 |
| 3.8 | 90 |
| 2.16 | 100 |

Fortsetzung Tabelle 5

| Verbindungen gemäß Beispiel | Wirkungsgrad in % bei mg Wirkstoff/ Liter Spritzbrühe 500 |
|---|---|
| 102.29 | 100 |
| 102.30 | 100 |
| 1.1 | 100 |
| 1.2 | 100 |
| 1.4 | 100 |
| 2.8 | 100 |
| 2.1 | 100 |
| 2.2 | 100 |
| 2.3 | 100 |
| 2.4 | 100 |
| 2.5 | 100 |
| 2.7 | 100 |
| 7.3 | 100 |
| 10.1 | 100 |
| 10.2 | 100 |
| 10.3 | 100 |
| 10.4 | 100 |
| 11.2 | 100 |
| 30.1 | 100 |
| 31.2 | 100 |
| 2.6 | 100 |
| unbehandelte, infizierte Pflanzen | 0 |

**Beispiel 6**

Etwa 5 Wochen alte Reispflanzen der Sorte "Ballila" wurden nach Vorspritzen mit 0,05 %iger Gelatinelösung mit den unten angegebenen Konzentrationen der beanspruchten Verbindungen behandelt. Nach Antrocknen des Spritzbelages wurden die Pflanzen mit einer Sporensuspension von Piricularia oryzae gleichmäßig inokuliert und 48 h in eine dunkel gehaltene Klimakammer mit einer Temperatur von 25 °C und 100 % rel. Luftfeuchte gestellt. Danach wurden die Reispflanzen in einem Gewächshaus bei einer Temperatur von 25 °C und 80 % rel. Luftfeuchte weiterkultiviert. Nach 5 Tagen erfolgte die Befallsauswertung. Der Wirkungsgrad der Prüfsubstanzen wurde prozentual zur unbehandelten, infizierten Kontrolle bonitiert und ist in Tabelle 6 wiedergegeben.

Tabelle 6

| Verbindungen gemäß Beispiel | Wirkungsgrad in % bei mg Wirkstoff/ Liter Spritzbrühe 500 |
|---|---|
| 9.17 | 100 |
| 2.34 | 100 |
| 2.41 | 100 |
| 2.43 | 100 |
| 2.45 | 100 |
| 2.46 | 100 |
| 2.48 | 100 |
| 2.47 | 100 |
| 2.49 | 100 |
| 7.18 | 100 |
| 2.51 | 90 |
| 7.20 | 90 |
| 7.8 | 100 |
| 7.7 | 90 |
| 7.10 | 90 |
| 7.11 | 100 |
| 7.12 | 100 |
| 8.2 | 100 |
| 8.5 | 100 |

**Fortsetzung Tabelle 6**

| Verbindungen gemäß Beispiel | Wirkungsgrad in % bei mg Wirkstoff/ Liter Spritzbrühe 500 |
|---|---|
| 7.13 | 100 |
| 7.14 | 100 |
| 7.15 | 100 |
| 7.16 | 100 |
| 2.19 | 100 |
| 3.6 | 90 |
| 2.11 | 100 |
| 2.21 | 100 |
| 3.4 | 90 |
| 2.14 | 90 |
| 3.8 | 100 |
| 2.33 | 90 |
| 2.9 | 100 |
| 120.1 | 90 |
| 120.6 | 90 |
| 6.9 | 100 |
| 102.11 | 100 |
| 102.21 | 100 |
| 102.16 | 100 |
| 102.22 | 100 |
| 102.17 | 100 |
| 102.23 | 100 |
| 102.18 | 100 |
| 102.13 | 90 |
| 102.32 | 100 |
| 102.29 | 100 |
| 1.1 | 100 |
| 1.2 | 100 |
| 2.1 | 100 |

**Fortsetzung Tabelle 6**

| Verbindungen gemäß Beispiel | Wirkungsgrad in % bei mg Wirkstoff/ Liter Spritzbrühe |
|---|---|
| | 500 |
| 2.2 | 100 |
| 7.1 | 100 |
| 7.3 | 100 |
| unbehandelte, infizierte Pflanzen | 0 |

**Beispiel 7**

Weizen der Sorte "Jubilar" wurde im 2-Blattstadium mit wäßrigen Suspensionen der beanspruchten Verbindungen tropfnaß behandelt.

Nach dem Antrocknen des Spritzbelages wurden die Pflanzen mit wäßrigen Sporensuspensionen von Puccinia recondita inokuliert. Die Pflanzen wurden für ca. 16 Stunden tropfnaß in eine Klimakammer 20 °C und ca. 100 % rel. Luftfeuchte gestellt. Anschließend wurden die infizierten Pflanzen in einem Gewächshaus bei einer Temperatur von 22 - 25 °C und 50 - 70 % rel. Luftfeuchte weiterkultiviert.

Nach einer Inkubationszeit von ca. 2 Wochen sporuliert der Pilz auf der gesamten Blattoberfläche der nicht behandelten Kontrollpflanzen, so daß eine Befallsauswertung der Versuchspflanzen vorgenommen werden kann. Der Wirkungsgrad der Prüfsubstanzen wurde prozentual zur unbehandelten, infizierten Kontrolle bonitiert und ist in Tabelle 7 wiedergegeben.

Tabelle 7

| Verbindungen gemäß Beispiel | Wirkungsgrad in % bei mg Wirkstoff/ Liter Spritzbrühe 500 |
|---|---|
| 9.16 | 90 |
| 9.17 | 100 |
| 9.18 | 100 |
| 2.45 | 90 |
| 2.49 | 90 |
| 7.8 | 100 |
| 7.11 | 90 |
| 7.12 | 100 |
| 8.5 | 100 |
| 7.14 | 100 |
| 7.15 | 100 |
| 7.16 | 100 |
| 2.8 | 100 |
| 2.19 | 100 |
| 3.6 | 100 |
| 2.11 | 100 |
| 2.21 | 90 |
| 2.14 | 90 |
| 2.16 | 90 |
| 5.8 | 100 |
| 2.9 | 100 |
| 3.5 | 90 |
| 120.5 | 90 |
| 6.9 | 90 |
| 102.11 | 100 |
| 102.17 | 100 |
| 102.10 | 100 |
| 102.33 | 100 |

**Fortsetzung Tabelle 7**

| Verbindungen gemäß Beispiel | Wirkungsgrad in % bei mg Wirkstoff/ Liter Spritzbrühe 500 |
|---|---|
| 1.1 | 100 |
| 1.2 | 100 |
| 2.7 | 100 |
| 2.1 | 100 |
| 2.2 | 100 |
| 7.1 | 100 |
| 31.3 | 100 |
| 7.3 | 100 |
| unbehandelte, infizierte Pflanzen | 0 |

**Beispiel 8**

Weinsämlinge der Sorten "Riesling/Ehrenfelder" wurden ca. 6 Wochen nach der Aussaat mit wäßrigen Suspensionen der beanspruchten Verbindung tropfnaß behandelt.

Nach dem Antrocknen des Spritzbelages wurden die Pflanzen mit einer Zoosporangiensuspension von Plasmopara viticola inokuliert und tropfnaß in eine Klimakammer mit 23 °C und 80 - 90 % rel. Luftfeuchte gestellt.

Nach einer Inkubationszeit von 7 Tagen wurden die Pflanzen über Nacht in die Klimakammer gestellt, um die Sporulation des Pilzes anzuregen. Anschließend erfolgte die Befallsauswertung. Der Wirkungsgrad der Prüfsubstanzen wurde prozentual zur unbehandelten, infizierten Kontrolle bonitiert und ist in Tabelle 8 wiedergegeben.

Tabelle 8

| Verbindung gemäß Beispiel | Wirkungagrad in % bei mg Wirkstoff/Liter Spritzbrühe 500 |
|---|---|
| 2.51 | 90 |
| 52.5 | 100 |
| 7.14 | 90 |
| 2.8 | 100 |
| 101.1 | 90 |
| 101.11 | 90 |
| 120.5 | 90 |
| 102.11 | 90 |
| 102.27 | 100 |
| 102.5 | 100 |
| 102.31 | 100 |
| 102.10 | 90 |
| 102.20 | 90 |
| 2.7 | 100 |
| unbehandelte, infizierte Pflanzen | 0 |

**Beispiel 9**

Weizenpflanzen der Sorte "Jubilar" wurden im 2-Blattstadium mit wäßrigen Suspensionen der in Tabelle 9 angegeben Präparate tropfnaß behandelt.

Nach dem Antrocknen des Spritzbelages wurden die Pflanzen mit einer wäßrigen Pyknosporen-Suspension von Leptosphaeria nodorum inokuliert und mehrere Stunden bei 100 % rel. Luftfeuchte in einer Klimakammer inkubiert. Bis zur Symptomausprägung wurden die Pflanzen im Gewächshaus bei ca. 90 % rel. Luftfeuchte weiterkultiviert.

Der Wirkungsgrad ist prozentual zur unbehandelten, infizierten Kontrolle ausgedrückt und wird in Tabelle 9 wiedergegeben.

Tabelle 9

| Verbindungen gemäß Beispiel | Wirkungsgrad in % bei mg Wirkstoff/ Liter Spritzbrühe 500 |
|---|---|
| 6.11 | 100 |
| 6.12 | 100 |
| 6.13 | 100 |
| 6.8 | 100 |
| 9.17 | 100 |
| 9.18 | 100 |
| 2.34 | 100 |
| 2.40 | 100 |
| 2.41 | 100 |
| 2.42 | 100 |
| 2.43 | 100 |
| 2.45 | 100 |
| 2.46 | 100 |
| 2.47 | 100 |
| 2.48 | 100 |
| 2.50 | 100 |
| 2.49 | 100 |
| 7.18 | 100 |
| 2.51 | 100 |
| 7.19 | 100 |
| 7.20 | 100 |
| 7.8 | 100 |
| 7.10 | 100 |
| 7.7 | 90 |
| 20.8 | 100 |
| 52.4 | 90 |

Fortsetzung Tabelle 9

| Verbindungen gemäß Beispiel | Wirkungsgrad in % bei mg Wirkstoff/ Liter Spritzbrühe 500 |
|---|---|
| 51.1 | 90 |
| 52.5 | 90 |
| 51.3 | 90 |
| 53.1 | 100 |
| 52.1 | 100 |
| 53.2 | 100 |
| 52.2 | 100 |
| 52.3 | 100 |
| 53.4 | 100 |
| 7.12 | 100 |
| 8.1 | 100 |
| 8.2 | 100 |
| 8.3 | 100 |
| 8.4 | 100 |
| 8.5 | 100 |
| 7.13 | 100 |
| 7.15 | 100 |
| 7.14 | 100 |
| 7.16 | 100 |
| 8.6 | 90 |
| 2.8 | 100 |
| 2.19 | 100 |
| 3.6 | 100 |
| 2.11 | 100 |
| 2.14 | 100 |
| 3.7 | 100 |
| 2.13 | 100 |
| 2.21 | 100 |
| 3.4 | 100 |

**Fortsetzung Tabelle 9**

| Verbindungen gemäß Beispiel | Wirkungsgrad in % bei mg Wirkstoff/ Liter Spritzbrühe 500 |
|---|---|
| 2.14 | 100 |
| 2.16 | 100 |
| 3.8 | 100 |
| 2.17 | 100 |
| 2.15 | 100 |
| 2.18 | 100 |
| 2.33 | 100 |
| 5.8 | 100 |
| 2.9 | 100 |
| 3.5 | 100 |
| 5.11 | 100 |
| 72.3 | 100 |
| 110.15 | 100 |
| 101.3 | 100 |
| 101.9 | 100 |
| 120.2 | 90 |
| 120.3 | 90 |
| 101.1 | 90 |
| 120.6 | 100 |
| 5.12 | 100 |
| 6.9 | 100 |
| 6.10 | 100 |
| 120.7 | 100 |
| 102.11 | 100 |
| 102.21 | 100 |
| 102.8 | 100 |
| 102.16 | 100 |
| 102.22 | 100 |
| 102.17 | 100 |

Fortsetzung Tabelle 9

| Verbindungen gemäß Beispiel | Wirkungsgrad in % bei mg Wirkstoff/ Liter Spritzbrühe 500 |
|---|---|
| 102.23 | 100 |
| 102.4 | 90 |
| 102.18 | 100 |
| 102.3 | 100 |
| 102.19 | 100 |
| 102.5 | 90 |
| 102.6 | 100 |
| 102.31 | 100 |
| 102.9 | 100 |
| 102.14 | 100 |
| 102.32 | 100 |
| 102.33 | 100 |
| 102.29 | 100 |
| 102.30 | 100 |
| 1.1 | 100 |
| 1.2 | 100 |
| 1.3 | 100 |
| 1.4 | 100 |
| 2.7 | 100 |
| 2.1 | 100 |
| 2.38 | 100 |
| 2.2 | 100 |
| 7.1 | 100 |
| 7.3 | 100 |
| 10.3 | 100 |
| 10.2 | 100 |
| 10.4 | 100 |
| 11.1 | 100 |
| 11.2 | 100 |

**Fortsetzung Tabelle 9**

| Verbindungen gemäß Beispiel | Wirkungsgrad in % bei mg Wirkstoff/ Liter Spritzbrühe |
|---|---|
| | 500 |
| 11.3 | 100 |
| 31.2 | 100 |
| unbehandelte, infizierte Pflanzen | 0 |

**Beispiel 10**

Gerstenpflanzen der Sorte "Igri" wurden im 2-Blattstadium mit einer wäßrigen Suspension der beanspruchten Verbindungen tropfnaß behandelt.

Nach dem Antrocknen des Spritzbelages wurden die Pflanzen mit wäßrigen Sporensuspensionen von Pyrenophora teres inokuliert und für 16 h in einer Klimakammer bei 100 % rel. Luftfeuchte inkubiert. Anschließend wurden die infizierten Pflanzen im Gewächshaus bei 25 °C und 80 % rel. Luftfeuchte weiterkultiviert.

Ca. 1 Woche nach Inokulation wurde der Befall ausgewertet. Der Wirkungsgrad der Prüfsubstanzen wurde prozentual zur unbehandelten, infizierten Kontrolle bonitiert und ist in Tabelle 10 wiedergegeben.

Tabelle 10

| Verbindungen gemäß Beispiel | Wirkungsgrad in % bei mg Wirkstoff/ Liter Spritzbrühe 500 |
|---|---|
| 6.11 | 90 |
| 6.12 | 100 |
| 6.13 | 100 |
| 9.17 | 90 |
| 9.18 | 100 |
| 2.34 | 100 |
| 2.40 | 100 |
| 2.41 | 90 |
| 2.42 | 90 |
| 2.43 | 90 |
| 2.46 | 100 |
| 2.48 | 100 |
| 2.49 | 100 |
| 7.18 | 100 |
| 2.51 | 100 |
| 7.19 | 100 |
| 7.11 | 90 |
| 52.5 | 100 |
| 51.3 | 100 |
| 7.12 | 90 |
| 8.1 | 90 |
| 8.2 | 100 |
| 8.3 | 90 |
| 7.13 | 100 |
| 7.15 | 90 |
| 7.14 | 100 |
| 7.16 | 90 |
| 2.8 | 100 |
| 2.19 | 100 |

Fortsetzung Tabelle 10

| Verbindungen gemäß Beispiel | Wirkungsgrad in % bei mg Wirkstoff/ Liter Spritzbrühe 500 |
|---|---|
| 3.6 | 90 |
| 2.11 | 90 |
| 2.14 | 100 |
| 2.13 | 100 |
| 2.21 | 100 |
| 3.4 | 100 |
| 2.14 | 100 |
| 3.8 | 100 |
| 2.16 | 90 |
| 2.15 | 100 |
| 2.18 | 100 |
| 2.33 | 100 |
| 5.11 | 100 |
| 2.9 | 90 |
| 101.1 | 100 |
| 101.3 | 100 |
| 101.5 | 100 |
| 101.4 | 90 |
| 120.2 | 100 |
| 120.3 | 100 |
| 120.4 | 100 |
| 6.10 | 100 |
| 6.14 | 100 |
| 5.12 | 90 |
| 102.21 | 100 |
| 102.22 | 100 |
| 102.3 | 100 |
| 102.23 | 100 |
| 120.8 | 90 |

**Fortsetzung Tabelle 10**

| Verbindungen gemäß Beispiel | Wirkungsgrad in % bei mg Wirkstoff/ Liter Spritzbrühe 500 |
|---|---|
| 102.19 | 100 |
| 102.27 | 100 |
| 102.6 | 90 |
| 102.15 | 100 |
| 102.31 | 100 |
| 102.9 | 100 |
| 102.32 | 100 |
| 102.29 | 100 |
| 102.30 | 100 |
| 2.7 | 100 |
| 7.1 | 100 |
| 10.3 | 100 |
| 10.2 | 100 |
| 11.2 | 100 |
| 11.3 | 100 |
| unbehandelte, infizierte Pflanzen | 0 |

**Beispiel 11**

Tomatenpflanzen der Sorte "Rheinlands Ruhm" wurden im 3 - 4 Blattstadium mit wäßrigen Suspensionen der beanspruchten Verbindungen gleichmäßig tropfnaß benetzt.

Nach dem Antrocknen wurden die Pflanzen mit einer Zoosporangien-Suspension von Phytophthora infestans inokuliert und für 2 Tage unter optimalen Infektionsbedingungen in einer Klimakammer gehalten. Danach wurden die Pflanzen bis zur Symptomausprägung im Gewächshaus weiterkultiviert.

Die Befallsbonitur erfolgte ca. 1 Woche nach Inokulation. Der Wirkungsgrad der Prüfsubstanzen wurde prozentual zur unbehandelten, infizierten Kontrolle bonitiert und ist in Tabelle 11 wiedergegeben.

Tabelle 11

| Verbindungen gemäß Beispiel | Wirkungsgrad in % bei mg Wirkstoff/Liter Spritzbrühe 500 |
|---|---|
| 2.34 | 90 |
| 2.40 | 100 |
| 2.41 | 100 |
| 2.49 | 100 |
| 7.18 | 100 |
| 7.8 | 90 |
| 7.11 | 90 |
| 8.1 | 100 |
| 7.12 | 90 |
| 8.2 | 100 |
| 2.19 | 90 |
| 2.13 | 100 |
| 2.21 | 100 |
| 2.16 | 90 |
| 2.18 | 90 |
| 2.9 | 100 |
| 101.1 | 90 |
| 101.5 | 90 |
| 102.5 | 90 |
| 102.33 | 90 |
| 10.3 | 100 |
| 10.2 | 100 |
| 10.4 | 100 |
| unbehandelte, infizierte Pflanzen | 0 |

**Patentansprüche**

**1.** Verbindungen der Formel I

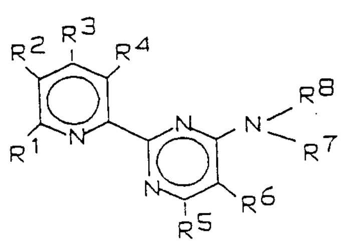

(I),

worin

$R^1$ = Wasserstoff, $(C_1$-$C_6)$Alkyl, $(C_1$-$C_4)$Alkoxy-$(C_1$-$C_4)$alkyl, $(C_1$-$C_4)$Alkylthio-$(C_1$-$C_4)$alkyl, $(C_2$-$C_6)$Alkenyl, $(C_2$-$C_6)$Alkinyl,$(C_3$-$C_7)$Cycloalkyl, $(C_3$-$C_7)$Cycloalkyl-$(C_1$-$C_4)$alkyl, wobei die beiden letztgenannten Reste im Cycloalkylteil bis zu dreifach durch $(C_1$-$C_4)$Alkyl substituiert sein können, eine Gruppe $R^7R^8N$-$(C_1$-$C_4)$alkyl, Phenyl, Phenoxy-$(C_1$-$C_4)$alkyl, Phenylmercapto-$(C_1$-$C_4)$alkyl, Phenyl-$(C_1$-$C_4)$alkyl, Phenoxy-phenoxy-$(C_1$-$C_4)$alkyl, wobei die fünf letztgenannten Reste im Phenylteil bis zu dreifach durch Halogen, Nitro, Cyano, $(C_1$-$C_4)$Alkyl, $(C_1$-$C_4)$Alkoxy, $(C_1$-$C_4)$Alkylthio, $(C_1$-$C_4)$Haloalkyl oder $(C_1$-$C_4)$Haloalkoxy substituiert sein können,

$R^2$, $R^3$, $R^4$ = unabhängig voneinander Wasserstoff, $(C_1$-$C_6)$Alkyl, Phenyl, wobei der Phenylrest bis zu dreifach durch Halogen, Nitro, Cyano, $(C_1$-$C_4)$Alkyl, $(C_1$-$C_4)$Alkoxy, $(C_1$-$C_4)$-Alkylthio, $(C_1$-$C_4)$Haloalkyl oder $(C_1$-$C_4)$Haloalkoxy substituiert sein kann,

$R^5$ = Wasserstoff, $(C_1-C_6)$Alkyl, $(C_3-C_7)$Cycloalkyl, $(C_3-C_7)$Cycloalkyl-$(C_1-C_4)$alkyl, wobei die beiden letztgenannten Reste im Cycloalkylteil bis zu dreifach durch $(C_1-C_4)$-Alkyl substituiert sein können, $(C_1-C_4)$Haloalkyl, $(C_1-C_4)$Alkoxy, $(C_1-C_4)$Alkylthio, $(C_1-C_4)$Alkoxy-$(C_1-C_4)$alkyl, eine Gruppe $R^7R^8N$-, $(C_1-C_4)$Alkylthio-$(C_1-C_4)$alkyl, eine Gruppe $R^7R^8N$-$(C_1-C_4)$alkyl, Halogen, $(C_2-C_6)$Alkenyl, $(C_2-C_6)$Alkinyl, Phenyl, Phenoxy, Phenyl$(C_1-C_4)$alkyl, Phenoxy-$(C_1-C_4)$alkyl, Phenylmercapto-$(C_1-C_4)$alkyl, Phenylmercapto, Phenyl-$(C_1-C_4)$alkoxy oder Phenyl-$(C_1-C_4)$alkylthio, wobei die acht letztgenannten Reste im Phenylteil bis zu dreifach durch Halogen, Nitro, Cyano, $(C_1-C_4)$Alkyl, $(C_1-C_4)$Alkoxy, $(C_1-C_4)$Alkylthio, $(C_1-C_4)$Haloalkyl oder $(C_1-C_4)$Haloalkoxy substituiert sein können;

$R^6$ = Wasserstoff, $(C_1-C_4)$Alkyl, $(C_1-C_4)$Alkoxy, $(C_2-C_6)$Alkenyloxy, $(C_2-C_6)$Alkinyloxy, $(C_1-C_4)$Alkylthio, Halogen, Phenyl, wobei der Phenylrest bis zu dreifach durch Halogen, Nitro, Cyano, $(C_1-C_4)$Alkyl, $(C_1-C_4)$Alkoxy, $(C_1-C_4)$Alkylthio, $(C_1-C_4)$-Haloalkyl oder $(C_1-C_4)$Haloalkoxy substituiert sein kann, oder

$R^5$ und $R^6$ bilden zusammen eine Polymethylenkette der Formel $-(CH_2)_m-$ mit m = 3 - 4

und

$R^7$ $(C_1-C_6)$Alkyl, $(C_1-C_4)$Alkoxy-$(C_1-C_6)$-Alkyl, Hydroxy-$(C_1-C_6)$Alkyl, $(C_1-C_4)$Alkylthio-$(C_1-C_6)$Alkyl, $R^9R^{10}N$-$(C_1-C_6)$Alkyl, $(C_3-C_6)$Alkenyl, $(C_3-C_6)$Alkinyl, $(C_3-C_7)$Cycloalkyl, $(C_3-C_7)$Cycloalkyl-$(C_1-C_4)$alkyl, wobei die beiden letztgenannten Reste im Cycloalkylteil bis zu dreifach durch $(C_1-C_4)$Alkyl substituiert sein können; Formyl, Phenyl, Phenyl-$(C_1-C_4)$alkyl, wobei die beiden letztgenannten Reste im Phenylteil bis zu dreifach durch Halogen, Nitro, Cyano, $(C_1-C_4)$Alkyl, $(C_1-C_4)$-Alkoxy, $(C_1-C_4)$Alkylthio, $(C_1-C_4)$-Haloalkyl oder $(C_1-C_4)$Haloalkoxy substituiert sein können;

bedeuten;

$R^8$ Wasserstoff bedeutet oder wie $R^7$ definiert ist;

oder beide Reste $R^7$, $R^8$ stehen zusammen mit dem Stickstoffatom, an das sie gebunden sind, für einen unsubstituierten oder bis zu vierfach mit $(C_1-C_4)$-Alkyl substituierten 5- bis 7-gliedrigen, gesättigten oder ungesättigten Heterocyclus mit 1 bis 3 gleichen oder verschiedenen Heteroatomen, worin die Heteroatome für Stickstoff, Sauerstoff und/oder Schwefel stehen;

$R^9$, $R^{10}$ = unabhängig voneinander Wasserstoff, $(C_1-C_6)$Alkyl, $(C_3-C_6)$Alkenyl, $(C_3-C_6)$Alkinyl, $(C_3-C_7)$Cycloalkyl, $(C_3-C_7)$Cycloalkyl-$(C_1-C_4)$alkyl, wobei die beiden letztgenannten Reste im Cycloalkylteil bis zu dreifach durch $(C_1-C_4)$Alkyl substituiert sein können; Formyl, Phenyl, Phenyl$(C_1-C_4)$alkyl, wobei die beiden letztgenannten Reste im Phenylteil bis zu dreifach durch Halogen, Nitro, Cyano, $(C_1-C_4)$Alkyl, $(C_1-C_4)$Alkoxy, $(C_1-C_4)$Alkylthio, $(C_1-C_4)$Haloalkyl oder $(C_1-C_4)$Haloalkoxy substituiert sein können;

oder beide Reste $R^9$, $R^{10}$ stehen zusammen mit dem Stickstoffatom, an das sie gebunden sind, für einen unsubstituierten oder bis zu vierfach mit $(C_1-C_4)$-Alkyl substituierten 5- bis 7-gliedrigen, gesättigten oder ungesättigten Heterocyclus mit 1 bis 3 gleichen oder verschiedenen Heteroatomen, worin die Heteroatome für Stickstoff, Sauerstoff und/oder Schwefel stehen; bedeuten, sowie deren Säureadditionssalze;

mit der Maßgabe, daß

die Reste $R^1$ - $R^8$ nicht gleichzeitig folgende Bedeutungen haben:

a) $R^1$, $R^2$, $R^3$, $R^4$, $R^6$ und $R^8$ jeweils = Wasserstoff, $R^5$ = Methyl, und $R^7$ = 2-(N,N-Dimethylamino)-ethyl oder 3-(N,N-Dimethylamino)-propyl; oder

b) $R^1$, $R^2$, $R^3$, $R^4$ und $R^6$ jeweils = Wasserstoff, $R^7$ und $R^8$ jeweils = Methyl, und $R^5$ = Chlor oder Ethylmercapto.

**2.** Verbindungen der Formel I gemäß Anspruch 1, worin

$R^1$ = Wasserstoff, $(C_1-C_6)$Alkyl, Phenyl, Phenyl-$(C_1-C_2)$-alkyl, Phenoxy-phenoxy-$(C_1-C_2)$-alkyl, Phenoxy-$(C_1-C_2)$-alkyl, wobei die vier letztgenannten Reste im Phenylteil bis zu dreifach durch Halogen oder $(C_1-C_4)$Alkyl substituiert sein können; $(C_1-C_3)$Alkoxy-$(C_1-C_2)$alkyl,

$R^2$, $R^3$ = unabhängig voneinander Wasserstoff, $(C_1-C_3)$Alkyl, Phenyl, wobei der Phenylrest bis zu dreifach durch Halogen oder $(C_1-C_4)$Alkyl substituiert sein kann,

$R^4$ = Wasserstoff,

$R^5$ = Wasserstoff, $(C_1-C_6)$Alkyl, $(C_3-C_6)$Cycloalkyl, $(C_5-C_6)$Cycloalkyl-$(C_1-C_3)$alkyl, Halogen,

Phenyl, Phenyl-($C_1$-$C_2$)alkyl, wobei die beiden letztgenannten Reste im Phenylteil unsubstituiert oder bis zu dreifach durch Halogen, ($C_1$-$C_4$)Alkyl oder ($C_1$-$C_4$)Alkoxy substituiert sein können,

$R^6$ = Wasserstoff, ($C_1$-$C_4$)Alkyl, Halogen, Phenyl, ($C_1$-$C_3$)Alkoxy oder

$R^5$ und $R^6$ bilden zusammen eine Polymethylenkette der Formel $-(CH_2)_m-$ mit m = 3 - 4 und

$R^7$ ($C_1$-$C_6$)Alkyl, ($C_1$-$C_4$)Alkoxy-($C_1$-$C_6$)-Alkyl, Hydroxy-($C_1$-$C_6$)Alkyl, ($C_1$-$C_4$)Alkylthio-($C_1$-$C_6$)Alkyl, $R^9R^{10}$N-($C_1$-$C_6$)Alkyl, ($C_3$-$C_4$)Alkenyl, ($C_3$-$C_4$)Alkinyl, ($C_3$-$C_6$)Cycloalkyl, ($C_3$-$C_6$)Cycloalkyl-($C_1$-$C_3$)alkyl, wobei die beiden letztgenannten Reste im Cycloalkylteil bis zu zweifach durch ($C_1$-$C_2$)Alkyl substituiert sein können; Formyl, Phenyl, Phenyl-($C_1$-$C_2$)alkyl, wobei die beiden letztgenannten Reste im Phenylteil bis zu zweifach durch Halogen, ($C_1$-$C_3$)Alkyl, ($C_1$-$C_3$)Alkoxy, Trifluormethyl oder Trichlormethyl substituiert sein können; bedeuten;

$R^8$ Wasserstoff bedeutet oder wie $R^7$ definiert ist, oder

beide Reste $R^7$, $R^8$ stehen zusammen mit dem Stickstoffatom, an das sie gebunden sind, für einen unsubstituierten oder bis zu zweifach mit ($C_1$-$C_3$)-Alkyl substituierten 5- bis 7-gliedrigen gesättigten oder ungesättigten Heterocyclus mit 1 oder 2 gleichen oder verschiedenen Heteroatomen Stickstoff und/oder Sauerstoff;

$R^9$, $R^{10}$ = unabhängig voneinander Wasserstoff, ($C_1$-$C_6$)Alkyl, ($C_3$-$C_6$)Alkenyl, ($C_3$-$C_6$)Alkinyl, ($C_3$-$C_7$)Cycloalkyl, ($C_3$-$C_7$)Cycloalkyl-($C_1$-$C_4$)alkyl, wobei die beiden letztgenannten Reste im Cycloalkylteil bis zu dreifach durch ($C_1$-$C_4$)Alkyl substituiert sein können; Formyl, Phenyl, Phenyl($C_1$-$C_4$)alkyl, wobei die beiden letztgenannten Reste im Phenylteil bis zu dreifach durch Halogen, Nitro, Cyano, ($C_1$-$C_4$)Alkyl, ($C_1$-$C_4$)Alkoxy, ($C_1$-$C_4$)Alkylthio, ($C_1$-$C_4$)Haloalkyl oder ($C_1$-$C_4$)Haloalkoxy substituiert sein können;

oder beide Reste $R^9$, $R^{10}$ stehen zusammen mit dem Stickstoffatom, an das sie gebunden sind, für einen unsubstituierten oder bis zu vierfach mit ($C_1$-$C_4$)-Alkyl substituierten 5- bis 7-gliedrigen, gesattigten oder ungesättigten Heterocyclus mit 1 bis 3 gleichen oder verschiedenen Heteroatomen Stickstoff, Sauerstoff und/oder Schwefel; bedeuten, sowie deren Säureadditionssalze.

**3.** Verfahren zur Herstellung von Verbindungen der Formel I gemäß Anspruch 1 oder 2, dadurch gekennzeichnet, daß man eine Verbindung der Formel II

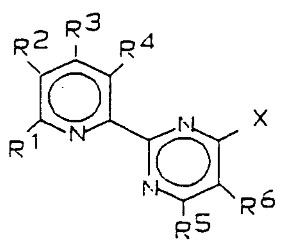

(II),

worin $R^1$ - $R^6$ die Bedeutungen wie in Formel I besitzen und X für Halogen steht, in Gegenwart einer Base mit einer Verbindung der Formel III

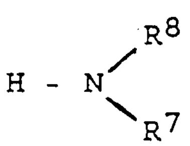

(III),

worin $R^7$ und $R^8$ die Bedeutungen wie in Formel I besitzen, umsetzt.

**4.** Fungizide Mittel, dadurch gekennzeichnet, daß sie eine wirksame Menge einer Verbindung der Formel I, worin $R^1$, $R^2$, $R^3$, $R^4$, $R^5$, $R^6$, $R^7$ und $R^8$ wie im Anspruch 1 oder 2 definiert sind und $R^7$ außerdem Wasserstoff bedeuten kann, und worin die die Reste $R^1$ - $R^8$ betreffende Maßgabe am Schluß des Anspruchs 1 ohne Gültigkeit ist, neben geeigneten Formulierungshilfsmitteln enthält.

5. Verwendung von Verbindungen der Formel I, worin $R^1$, $R^2$, $R^3$, $R^4$, $R^5$, $R^6$, $R^7$ und $R^8$ wie im Anspruch 1 oder 2 definiert sind und $R^7$ außerdem Wasserstoff bedeuten kann, und worin die die Reste $R^1$ - $R^8$ betreffende Maßgabe am Schluß des Anspruchs 1 ohne Gültigkeit ist, zur Bekämpfung von Schadpilzen.

6. Verfahren zur Bekämpfung von Schadpilzen, dadurch gekennzeichnet, daß man auf die von ihnen befallenen Pflanzen, Flächen oder Substrate eine wirksame Menge einer Verbindung der Formel I, worin $R^1$, $R^2$, $R^3$, $R^4$, $R^5$, $R^6$, $R^7$ und $R^8$ wie im Anspruch 1 oder 2 definiert sind und $R^7$ außerdem Wasserstoff bedeuten kann, und worin die die Reste $R^1$ - $R^8$ betreffende Maßgabe am Schluß des Anspruchs 1 ohne Gültigkeit ist, appliziert.

**Claims**

1. A compound of the formula I

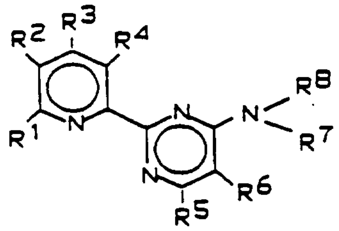

(I),

where

| | |
|---|---|
| $R^1$ is | hydrogen, $(C_1$-$C_6)$alkyl, $(C_1$-$C_4)$alkoxy-$(C_1$-$C_4)$alkyl, $(C_1$-$C_4)$alkylthio-$(C_1$-$C_4)$alkyl, $(C_2$-$C_6)$alkenyl, $(C_2$-$C_6)$alkynyl, $(C_3$-$C_7)$cycloalkyl or $(C_3$-$C_7)$cycloalkyl-$(C_1$-$C_4)$alkyl, where the last two radicals can be up to trisubstituted in the cycloalkyl moiety by $(C_1$-$C_4)$alkyl, or is a group $R^7R^8N$-$(C_1$-$C_4)$alkyl, phenyl, phenoxy-$(C_1$-$C_4)$alkyl, phenylmercapto-$(C_1$-$C_4)$alkyl, phenyl-$(C_1$-$C_4)$alkyl or phenoxyphenoxy-$(C_1$-$C_4)$alkyl, where the last five radicals can be up to trisubstituted in the phenyl moiety by halogen, nitro, cyano, $(C_1$-$C_4)$-alkyl, $(C_1$-$C_4)$alkoxy, $(C_1$-$C_4)$alkylthio, $(C_1$-$C_4)$-haloalkyl or $(C_1$-$C_4)$haloalkoxy, |
| $R^2$, $R^3$ and $R^4$ | independently of one another are hydrogen, $(C_1$-$C_6)$alkyl or phenyl, where the phenyl radical can be up to trisubstituted by halogen, nitro, cyano, $(C_1$-$C_4)$alkyl, $(C_1$-$C_4)$alkoxy, $(C_1$-$C_4)$alkylthio, $(C_1$-$C_4)$haloalkyl or $(C_1$-$C_4)$haloalkoxy, |
| $R^5$ is | hydrogen, $(C_1$-$C_6)$alkyl, $(C_3$-$C_7)$cycloalkyl or $(C_3$-$C_7)$cycloalkyl-$(C_1$-$C_4)$alkyl, where the last two radicals can be up to trisubstituted in the cycloalkyl moiety by $(C_1$-$C_4)$alkyl, or is $(C_1$-$C_4)$haloalkyl, $(C_1$-$C_4)$alkoxy, $(C_1$-$C_4)$alkylthio, $(C_1$-$C_4)$alkoxy-$(C_1$-$C_4)$alkyl, a group $R^7R^8N$-, $(C_1$-$C_4)$alkylthio-$(C_1$-$C_4)$alkyl, a group $R^7R^8N$-$(C_1$-$C_4)$-alkyl, halogen, $(C_2$-$C_6)$alkenyl, $(C_2$-$C_6)$alkynyl, phenyl, phenoxy, phenyl$(C_1$-$C_4)$-alkyl, phenoxy-$(C_1$-$C_4)$alkyl, phenylmercapto-$(C_1$-$C_4)$alkyl, phenylmercapto, phenyl-$(C_1$-$C_4)$alkoxy or phenyl-$(C_1$-$C_4)$alkylthio, ,where the last eight radicals can be up to trisubstituted in the phenyl moiety by halogen, nitro, cyano, $(C_1$-$C_4)$alkyl, $(C_1$-$C_4)$alkoxy, $(C_1$-$C_4)$alkylthio, $(C_1$-$C_4)$haloalkyl or $(C_1$-$C_4)$haloalkoxy; |
| $R^6$ is | hydrogen, $(C_1$-$C_4)$alkyl, $(C_1$-$C_4)$alkoxy, $(C_2$-$C_6)$-alkenyloxy, $(C_2$-$C_6)$alkynyloxy, $(C_1$-$C_4)$alkylthio, halogen or phenyl, where the phenyl radical can be up to trisubstituted by halogen, nitro, cyano, $(C_1$-$C_4)$alkyl, $(C_1$-$C_4)$alkoxy, $(C_1$-$C_4)$alkylthio, $(C_1$-$C_4)$haloalkyl or $(C_1$-$C_4)$haloalkoxy, or |
| $R^5$ and $R^6$ | together form a polymethylene chain of the formula $-(CH_2)_m-$ with m being 3 - 4 |

and

| | |
|---|---|
| $R^7$ is | $(C_1$-$C_6)$alkyl, $(C_1$-$C_4)$alkoxy-$(C_1$-$C_6)$alkyl, hydroxy-$(C_1$-$C_6)$alkyl, $(C_1$-$C_4)$alkylthio-$(C_1$-$C_6)$alkyl, $R^9R^{10}N$-$(C_1$-$C_6)$ alkyl, $(C_3$-$C_6)$alkenyl, $(C_3$-$C_6)$alkynyl, $(C_3$-$C_7)$cycloalkyl or $(C_3$-$C_7)$cycloalkyl-$(C_1$-$C_4)$alkyl, where the last two radicals can be up to trisubstituted in the cycloalkyl moiety by $(C_1$-$C_4)$alkyl; or is formyl, phenyl or phenyl-$(C_1$-$C_4)$alkyl, where the last two radicals can be up to trisubstituted in the phenyl moiety by halogen, nitro, cyano, $(C_1$-$C_4)$alkyl, $(C_1$-$C_4)$alkoxy, $(C_1$-$C_4)$alkylthio, $(C_1$-$C_4)$haloalkyl |

or $(C_1-C_4)$haloalkoxy;

$R^8$ is hydrogen or is defined as $R^7$;

or the two radicals $R^7$ and $R^8$ together with the nitrogen atom to which they are bonded are a 5- to 7-membered, saturated or unsaturated heterocycle which is unsubstituted or up to tetrasubstituted by $(C_1-C_4)$-alkyl and which has 1 to 3 identical or different hetero atoms, where the hetero atoms are nitrogen, oxygen and/or sulfur;

$R^9$ and $R^{10}$ independently of one another are hydrogen, $(C_1-C_6)$alkyl, $(C_3-C_6)$alkenyl, $(C_3-C_6)$-alkynyl, $(C_3-C_7)$cycloalkyl or $(C_3-C_7)$cycloalkyl-$(C_1-C_4)$alkyl, where the last two radicals can be up to trisubstituted in the cycloalkyl moiety by $(C_1-C_4)$alkyl; or are formyl, phenyl, phenyl$(C_1-C_4)$alkyl, where the last two radicals can be up to trisubstituted in the phenyl moiet y by halogen, nitro, cyano, $(C_1-C_4)$alkyl, $(C_1-C_4)$alkoxy, $(C_1-C_4)$alkylthio, $(C_1-C_4)$haloalkyl or $(C_1-C_4)$haloalkoxy;

or the two radicals $R^9$ and $R^{10}$ together with the nitrogen atom to which they are bonded are a 5- to 7-membered, saturated or unsaturated heterocycle which is unsubstituted or up to tetrasubstituted by $(C_1-C_4)$-alkyl and which has 1 to 3 identical or different hetero atoms, where the hetero atoms are nitrogen, oxygen and/or sulfur; and the acid addition salts thereof;

with the proviso that

the radical $R^1$ - $R^8$ are not simultaneously as defined below:

a) $R^1$, $R^2$, $R^3$, $R^4$, $R^6$ and $R^8$ are in each case hydrogen, $R^5$ is methyl and $R^7$ is 2-(N,N-dimethylamino) ethyl or 3-(N,N-dimethylamino)propyl; or

b) $R^1$, $R^2$, $R^3$, $R^4$ and $R^6$ are in each case hydrogen, $R^7$ and $R^8$ are in each case methyl and $R^5$ is chlorine or ethylmercapto.

**2.** A compound of the formula I as claimed in claim 1, where

$R^1$ is hydrogen, $(C_1-C_6)$alkyl, phenyl, phenyl-$(C_1-C_2)$alkyl, phenoxyphenoxy-$(C_1-C_2)$alkyl or phenoxy-$(C_1-C_2)$alkyl, where the last four radicals can be up to trisubstituted in the phenyl moiety by halogen or $(C_1-C_4)$alkyl; or is $(C_1-C_3)$alkoxy-$(C_1-C_2)$alkyl,

$R^2$ and $R^3$ independently of one another are hydrogen, $(C_1-C_3)$alkyl or phenyl, where the phenyl radical can be up to trisubstituted by halogen or $(C_1-C_4)$alkyl,

$R^4$ is hydrogen,

$R^5$ is hydrogen, $(C_1-C_6)$alkyl, $(C_3-C_6)$cycloalkyl, $(C_5-C_6)$cycloalkyl-$(C_1-C_3)$alkyl, halogen, phenyl or phenyl-$(C_1-C_2)$alkyl, where the last two radicals can be unsubstituted or up to trisubstituted in the phenyl moiety by halogen, $(C_1-C_4)$alkyl or $(C_1-C_4)$alkoxy,

$R^6$ is hydrogen, $(C_1-C_4)$alkyl, halogen, phenyl or $(C_1-C_3)$alkoxy, or

$R^5$ and $R^6$ together form a polymethylene chain of the formula $-(CH_2)_m-$ with m being 3 - 4, and

$R^7$ is $(C_1-C_6)$alkyl, $(C_1-C_4)$alkoxy-$(C_1-C_6)$alkyl, hydroxy-$(C_1-C_6)$alkyl, $(C_1-C_4)$alkylthio-$(C_1-C_6)$alkyl, $R^9R^{10}N$-$(C_1-C_6)$alkyl, $(C_3-C_4)$alkenyl, $(C_3-C_4)$alkynyl, $(C_3-C_6)$cycloalkyl or $(C_3-C_6)$cycloalkyl-$(C_1-C_3)$alkyl, where the last two radicals can be up to disubstituted in the cycloalkyl moiety by $(C_1-C_2)$alkyl; or is formyl, phenyl or phenyl-$(C_1-C_2)$alkyl, where the last two radicals can be up to disubstituted in the phenyl moiety by halogen, $(C_1-C_3)$alkyl, $(C_1-C_3)$alkoxy, trifluoromethyl or trichloromethyl;

$R^8$ is hydrogen or is defined as $R^7$, or

the two radicals $R^7$ and $R^8$ together with the nitrogen atom to which they are bonded are a 5- to 7-membered, saturated or unsaturated heterocycle which is unsubstituted or up to disubstituted by $(C_1-C_3)$alkyl and which has 1 or 2 identical or different hetero atoms, nitrogen and/or oxygen;

$R^9$ and $R^{10}$ independently of one another are hydrogen, $(C_1-C_6)$alkyl, $(C_3-C_6)$alkenyl, $(C_3-C_6)$-alkynyl, $(C_3-C_7)$cycloalkyl or $(C_3-C_7)$cycloalkyl-$(C_1-C_4)$alkyl, where the last two radicals can be up to trisubstituted in the cycloalkyl moiety by $(C_1-C_4)$alkyl; or are formyl, phenyl, phenyl$(C_1-C_4)$alkyl, where the last two radicals can be up to trisubstituted in the phenyl moiety by halogen, nitro, cyano, $(C_1-C_4)$alkyl, $(C_1-C_4)$alkoxy, $(C_1-C_4)$alkylthio, $(C_1-C_4)$haloalkyl or $(C_1-C_4)$haloalkoxy;

or the two radicals $R^9$ and $R^{10}$ together with the nitrogen atom to which they are bonded are a 5- to 7-membered, saturated or unsaturated heterocycle which is unsubstituted or up to tetrasubstituted by $(C_1-C_4)$-alkyl and which has 1 to 3 identical or different hetero atoms, nitrogen, oxygen and/or sulfur; and the acid addition salts thereof.

3. A process for the preparation of a compound of the formula I as claimed in claim 1 or 2, which comprises reacting a compound of the formula II

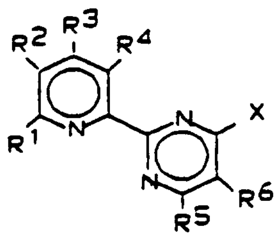

(II)

where $R^1$ - $R^6$ are as defined in formula I and X represents halogen, with a compound of the formula III

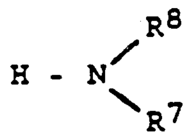

(III)

where $R^7$ and $R^8$ are as defined in formula I, in the presence of a base.

4. A fungicidal agent which comprises an effective amount of a compound of the formula I in which $R^1$, $R^2$, $R^3$, $R^4$, $R^5$, $R^6$, $R^7$ and $R^8$ are as defined in Claim 1 or 2 and $R^7$ can furthermore be hydrogen, and in which the proviso relating to the radicals $R^1$ - $R^8$ at the end of Claim 1 does not apply, in addition to suitable formulation auxiliaries.

5. The use of a compound of the formula I in which $R^1$, $R^2$, $R^3$, $R^4$, $R^5$, $R^6$, $R^7$ and $R^8$ are as defined in Claim 1 or 2 and $R^7$ can furthermore be hydrogen, and in which the proviso relating to the radicals $R^1$ - $R^8$ at the end of Claim 1 does not apply, for controlling harmful fungi.

6. A method of controlling harmful fungi, which comprises applying an effective amount of a compound of the formula I in which $R^1$, $R^2$, $R^3$, $R^4$, $R^5$, $R^6$, $R^7$ and $R^8$ are as defined in Claim 1 or 2 and $R^7$ can furthermore be hydrogen, and in which the proviso relating to the radicals $R^1$ - $R^8$ at the end of Claim 1 does not apply, to the plants, areas or substrates which are diseased with the fungi.

**Revendications**

1. Composés de formule I

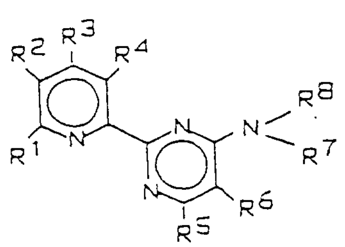

(I)

dans laquelle
$R^1$ est un hydrogène ou un radical alkyle en $C_1$-$C_6$, (alcoxy en $C_1$-$C_4$)-(alkyle en $C_1$-$C_4$), (alkyl en $C_1$-$C_4$)-thio-(alkyle en $C_1$-$C_4$), alcényle en $C_2$-$C_6$, alcynyle en $C_2$-$C_6$, cycloalkyle en $C_3$-$C_7$, (cycloalkyl en $C_3$-$C_7$)-(alkyle en $C_1$-$C_4$), ces deux derniers radicaux pouvant, dans le fragment cycloalkyle, être substitués jusqu'à trois fois par des substituants alkyle en $C_1$-$C_4$, un groupe $R^7R^8N$-(alkyle en $C_1$-$C_4$), phényle, phénoxy-(alkyle en $C_1$-$C_4$), phénylmercapto-(alkyle en $C_1$-$C_4$), phényl-(alkyle en $C_1$-$C_4$), phénoxyphénoxy-(alkyle en $C_1$-$C_4$), ces cinq derniers radicaux pouvant, dans le fragment phényle, être substitués jusqu'à trois fois par des substituants halogéno, nitro, cyano, alkyle en $C_1$-$C_4$, alcoxy en $C_1$-

$C_4$, alkylthio en $C_1$-$C_4$, halogénalkyle en $C_1$-$C_4$ ou halogénalcoxy en $C_1$-$C_4$,
$R^2$, $R^3$ et $R^4$, indépendamment l'un de l'autre, sont chacun un hydrogène ou un radical alkyle en $C_1$-$C_6$, ou le radical phényle, le radical phényle pouvant être substitue jusqu'à trois fois par des substituants halogéno, nitro, cyano, alkyle en $C_1$-$C_4$, alcoxy en $C_1$-$C_4$, alkylthio en $C_1$-$C_4$, halogénalkyle en $C_1$-$C_4$ ou halogénalcoxy en $C_1$-$C_4$,
$R^5$ est un hydrogène ou un radical alkyle en $C_1$-$C_6$, cycloalkyle en $C_3$-$C_7$, (cycloalkyl en $C_3$-$C_7$)-(alkyle en $C_1$-$C_4$), ces deux derniers radicaux pouvant, dans le fragment cycloalkyle, être substitués jusqu'à trois fois par des substituants alkyle en $C_1$-$C_4$ ; halogénalkyle en $C_1$-$C_4$, alcoxy en $C_1$-$C_4$, alkylthio en $C_1$-$C_4$, (alcoxy en $C_1$-$C_4$)-(alkyle en $C_1$-$C_4$), un groupe $R^7R^8N$-, (alkyl en $C_1$-$C_4$)-thio-(alkyle en $C_1$-$C_4$), un groupe $R^7R^8N$-(alkyle en $C_1$-$C_4$), halogéno, alcényle en $C_2$-$C_6$, alcynyle en $C_2$-$C_6$, phényle, phénoxy, phényl-(alkyle en $C_1$-$C_4$), phénoxy-(alkyle en $C_1$-$C_4$), phénylmercapto-(alkyle en $C_1$-$C_4$), phénylmercapto, phényl(alcoxy en $C_1$-$C_4$) ou phényl-(alkylthio en $C_1$-$C_4$), ces huit derniers radicaux pouvant être substitués dans le fragment phényle jusqu'à trois fois par des substituants halogéno, nitro, cyano, alkyle en $C_1$-$C_4$, alcoxy en $C_1$-$C_4$, alkylthio en $C_1$-$C_4$, halogénalkyle en $C_1$-$C_4$ ou halogénalcoxy en $C_1$-$C_4$ ;
$R^6$ est un hydrogène ou un radical alkyle en $C_1$-$C_4$, alcoxy en $C_1$-$C_4$, alcényloxy en $C_2$-$C_6$, alcynyloxy en $C_2$-$C_6$, alkylthio en $C_1$-$C_4$, halogéno, phényle, le radical phényle pouvant être substitué jusqu'à trois fois par des substituants halogéno, nitro, cyano, alkyle en $C_1$-$C_4$, alcoxy en $C_1$-$C_4$, alkylthio en $C_1$-$C_4$, halogénalkyle en $C_1$-$C_4$ ou halogénalcoxy en $C_1$-$C_4$, ou bien
$R^5$ et $R^6$ forment ensemble une chaîne polyméthylène de formule $-(CH_2)_m-$ où m = 3-4, et
$R^7$ est un radical alkyle en $C_1$-$C_6$, (alcoxy en $C_1$-$C_4$)-(alkyle en $C_1$-$C_6$), hydroxy-(alkyle en $C_1$-$C_6$), (alkyl en $C_1$-$C_4$)-thio-(alkyle en $C_1$-$C_6$), $R^9R^{10}N$-(alkyle en $C_1$-$C_6$), alcényle en $C_3$-$C_6$, alcynyle en $C_3$-$C_6$, cycloalkyle en $C_3$-$C_7$, (cycloalkyl en $C_3$-$C_7$)-(alkyle en $C_1$-$C_4$), ces deux derniers radicaux pouvant être substitués dans le fragment cycloalkyle jusqu'à trois fois par des substituants alkyle en $C_1$-$C_4$ ; formyle, phényle, phényl-(alkyle en $C_1$-$C_4$), ces deux derniers radicaux pouvant être substitués dans le fragment phényle jusqu'à trois fois par des substituants halogéno, nitro, cyano, alkyle en $C_1$-$C_4$, alcoxy en $C_1$-$C_4$, alkylthio en $C_1$-$C_4$, halogénalkyle en $C_1$-$C_4$ ou halogénalcoxy en $C_1$-$C_4$ ;
$R^8$ est un hydrogène ou tel que défini pour $R^7$ ;
ou bien les deux radicaux $R^7$ et $R^8$, avec l'atome d'azote auquel ils sont liés, représentent un radical hétérocyclique, non substitué, ou substitué jusqu'à quatre fois par des substituants alkyle en $C_1$-$C_4$, à 5 à 7 chaînons, saturé ou insaturé, ayant de 1 à 3 hétéroatomes identiques ou différents, les hétéroatomes étant l'azote, l'oxygène et/ou le soufre ;
$R^9$ et $R^{10}$, indépendamment l'un de l'autre, sont chacun un hydrogène ou un radical alkyle en $C_1$-$C_6$, alcényle en $C_3$-$C_6$, alcynyle en $C_3$-$C_6$, cycloalkyle en $C_3$-$C_7$, (cycloalkyl en $C_3$-$C_7$)-(alkyle en $C_1$-$C_4$), ces deux derniers radicaux pouvant être substitués dans le fragment cycloalkyle jusqu'à trois fois par des substituants alkyle en $C_1$-$C_4$ ; formyle, phényle, phényl-(alkyle en $C_1$-$C_4$), ces deux derniers radicaux pouvant être substitués dans le fragment phényle jusqu'à trois fois par des substituants halogéno, nitro, cyano, alkyle en $C_1$-$C_4$, alcoxy en $C_1$-$C_4$, alkylthio en $C_1$-$C_4$, halogénalkyle en $C_1$-$C_4$ ou halogénalcoxy en $C_1$-$C_4$ ; ou bien les deux radicaux $R^9$ et $R^{10}$, avec l'atome d'azote auquel ils sont liés, représentent un radical hétérocyclique, non substitué, ou substitué jusqu'à quatre fois par des substituants alkyle en $C_1$-$C_4$, à 5 à 7 chaînons, saturé ou insaturé, ayant de 1 à 3 hétéroatomes identiques ou différents, les hétéroatomes étant l'azote, l'oxygène et/ou le soufre ;
ainsi que leurs sels d'addition avec un acide,
du moment que les radicaux $R^1$ à $R^8$ n'ont pas simultanément les significations suivantes :

a) $R^1$, $R^2$, $R^3$, $R^4$, $R^5$, $R^6$ et $R^8$ représentent chacun un hydrogène, $R^5$ est le radical méthyle et $R^7$ est le radical 2-(N,N-diméthylamino)-éthyle ou 3-(N,N-diméthylamino)-propyle ; ou bien

b) $R^1$, $R^2$, $R^3$, $R^4$, $R^5$ et $R^6$ sont chacun un hydrogène, $R^7$ et $R^8$ sont chacun le radical méthyle, et $R^5$ est le radical chloro ou éthylmercapto.

**2.** Composés de formule I selon la revendication 1, dans lesquels
$R^1$ est un hydrogène ou un radical alkyle en $C_1$-$C_6$, phényle, phényl-(alkyle en $C_1$-$C_2$), phénoxyphénoxy-(alkyle en $C_1$-$C_2$), phénoxy-(alkyle en $C_1$-$C_2$), ces quatre derniers radicaux pouvant être substitués dans le fragment phényle jusqu'à trois fois par des substituants halogéno ou alkyle en $C_1$-$C_4$ ; ou encore (alcoxy en $C_1$-$C_3$)-(alkyle en $C_1$-$C_2$), $R^2$ et $R^3$, indépendamment l'un de l'autre, sont chacun un hydrogène ou un radical alkyle en $C_1$-$C_3$ ou phényle, le radical phényle pouvant être substitué jusqu'à trois fois par des substituants halogéno ou alkyle en $C_1$-$C_4$,
$R^4$ est un hydrogène,
$R^5$ est un hydrogène ou un radical alkyle en $C_1$-$C_6$, cycloalkyle en $C_3$-$C_6$, (cycloalkyl en $C_5$-$C_6$)-(alkyle

en $C_1$-$C_3$), halogéno, phényle, phényl-(alkyle en $C_1$-$C_2$), ces deux derniers radicaux pouvant, dans le fragment phényle, être non substitués ou encore substitués jusqu'à trois fois par des substituants halogéno, alkyle en $C_1$-$C_4$ ou alcoxy en $C_1$-$C_4$, $R^6$ est un hydrogène ou un radical alkyle en $C_1$-$C_4$, halogéno, phényle, alcoxy en $C_1$-$C_3$, ou bien
$R^5$ et $R^6$ forment ensemble une chaîne polyméthylène de formule $-(CH_2)_m-$ où m = 3-4, et
$R^7$ est un radical alkyle en $C_1$-$C_6$, (alcoxy en $C_1$-$C_4$)-(alkyle en $C_1$-$C_6$), hydroxy-(alkyle en $C_1$-$C_6$), (alkyl en $C_1$-$C_4$)-thio-(alkyle en $C_1$-$C_6$), $R^9R^{10}$N-(alkyle en $C_1$-$C_6$), alcényle en $C_3$-$C_4$, alcynyle en $C_3$-$C_4$, cycloalkyle en $C_3$-$C_6$, (cycloalkyl en $C_3$-$C_6$)-(alkyle en $C_1$-$C_3$), ces deux derniers radicaux pouvant être substitués dans le fragment cycloalkyle jusqu'à deux fois par des substituants alkyle en $C_1$-$C_2$ ; formyle, phényle, phényl-(alkyle en $C_1$-$C_2$), ces deux derniers radicaux pouvant être substitués dans le fragment phényle jusqu'à deux fois par des substituants halogéno, alkyle en $C_1$-$C_3$, alcoxy en $C_1$-$C_3$, trifluorométhyle ou trichlorométhyle ;
$R^8$ est un hydrogène ou est tel que défini pour $R^7$, ou bien les deux radicaux $R^7$ et $R^8$ forment ensemble, avec l'atome d'azote auquel ils sont liés, un radical hétérocyclique non substitué, ou substitué jusqu'à deux fois par des substituants alkyle en $C_1$-$C_3$, à 5 à 7 chaînons, saturé ou insaturé, ayant 1 ou 2 hétéroatomes identiques ou différents, qui sont l'azote et/ou l'oxygène ;
$R^9$ et $R^{10}$, indépendamment l'un de l'autre, sont chacun un hydrogène, un radical alkyle en $C_1$-$C_6$, alcényle en $C_3$-$C_6$, alcynyle en $C_3$-$C_6$, cycloalkyle en $C_3$-$C_7$, (cycloalkyl en $C_3$-$C_7$)-(alkyle en $C_1$-$C_4$), ces deux derniers radicaux pouvant être substitués dans le fragment cycloalkyle jusqu'à trois fois par des substituants alkyle en $C_1$-$C_4$ ; formyle, phényle, phényl-(alkyle en $C_1$-$C_4$), ces deux derniers radicaux pouvant être substitués dans le fragment phényle jusqu'à trois fois par des substituants halogéno, nitro, cyano, alkyle en $C_1$-$C_4$, alcoxy en $C_1$-$C_4$, alkylthio en $C_1$-$C_4$, halogénalkyle en $C_1$-$C_4$ ou halogénalcoxy en $C_1$-$C_4$ ; ou bien les deux radicaux $R^9$ et $R^{10}$, avec l'atome d'azote auquel ils sont liés, représentent un radical hétérocyclique, non substitué, ou substitué jusqu'à quatre fois par des substituants alkyle en $C_1$-$C_4$, à 5 à 7 chaînons, saturé ou insaturé, ayant de 1 à 3 hétéroatomes identiques ou différents, les hétéroatomes étant l'azote, l'oxygène et/ou le soufre ;
ainsi que leurs sels d'addition avec un acide.

**3.** Procédé pour préparer des composés de formule I selon la revendication 1 ou 2, caractérisé en ce qu'on fait réagir un composé de formule II

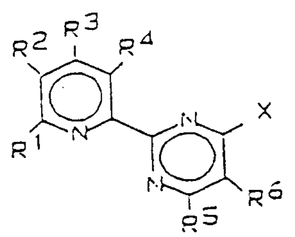

(II)

dans laquelle $R^1$ à $R^6$ ont les significations données pour la formule I et X est un halogène, en présence d'une base, avec un composé de formule III

$$H-N\begin{matrix}/R^8\\ \backslash R^7\end{matrix} \qquad (III)$$

dans laquelle $R^7$ et $R^8$ ont les significations données pour la formule I.

**4.** Produits fongicides, caractérisés en ce qu'ils contiennent une quantité active d'un composé de formule I dans laquelle $R^1$, $R^2$, $R^3$, $R^4$, $R^5$, $R^6$, $R^7$ et $R^8$ ont les définitions données dans la revendication 1 ou 2 et $R^7$ peut en outre être un hydrogène, et dans lesquels la condition relative aux radicaux $R^1$ à $R^8$ et se trouvant à la fin de la revendication 1 ne s'applique pas, et ce, en plus d'auxiliaires appropriés de formulation.

**5.** Utilisation de composés de formule I, dans laquelle $R^1$, $R^2$, $R^3$, $R^4$, $R^5$, $R^6$, $R^7$ et $R^8$ sont tels que définis dans la revendication 1 ou 2, et $R^7$ peut en outre être un hydrogène, et où la condition relative aux radicaux $R^1$ à $R^8$, à la fin de la revendication 1, ne s applique pas, pour la maîtrise des champignons nuisibles.

**6.** Procédé pour maîtriser les champignons nuisibles, caractérisé en ce qu'on applique sur les plants, aires ou substrats infestés par ces derniers, une quantité active d'un composé de formule I dans laquelle $R^1$, $R^2$, $R^3$, $R^4$, $R^5$, $R^6$, $R^7$ et $R^8$ sont tels que définis dans la revendication 1 ou 2 et $R^7$ peut en outre être un hydrogène, et où la condition relative aux radicaux $R^1$ à $R^8$ et se trouvant à la fin de la revendication 1 ne s'applique pas.